# EUROPEAN PATENT APPLICATION

(11) **EP 4 843 580 A1**
(43) Date of publication of application: **25.11.2026**
(21) Application number: 25741648.7
(22) Date of filing: 18.01.2025
(51) Int. Cl.: A61K 36/605, A61P 15/10, A61P 15/08

(54) **USE OF MULBERRY EXTRACT IN PREPARATION OF DRUG FOR TREATING AND/OR PREVENTING MALE INFERTILITY**

(30) Priority: 19.01.2024 CN 202410084560; 16.01.2025 CN 202510072973
(71) Applicant: Beijing Wehand-Bio Pharmaceutical Co., Ltd, Beijing 102600 (CN); Guangxi Wehand-Bio Pharmaceutical Co., Ltd, Hechi, Guangxi 546300 (CN)
(72) Inventor: LIU, Yuling, Beijing 102600 (CN); LIU, Yuanyuan, Beijing 102600 (CN); WANG, Xingang, Beijing 102600 (CN); LI, Huijuan, Beijing 102600 (CN); MA, Luyao, Beijing 102600 (CN); LIU, Dongdong, Beijing 102600 (CN); HU, Wangmin, Beijing 102600 (CN); FENG, Yu, Beijing 102600 (CN); HAN, Yanqi, Beijing 102600 (CN); YANG, Yanfang, Beijing 102600 (CN); LIU, Zhihua, Beijing 102600 (CN); ZHU, Xiangyang, Beijing 102600 (CN); CHEN, Yanmin, Beijing 102600 (CN); WANG, Tingting, Beijing 102600 (CN); ZOU, Yuanyuan, Beijing 102600 (CN); JIN, Yiqun, Beijing 102600 (CN); CAO, Haiyan, Beijing 102600 (CN)
(74) Representative: Bandpay & Greuter
(86) International application number: PCT/CN2025/073161
(87) International publication number: WO 2025/153086

(57) **Abstract**

Use of a mulberry extract in the preparation of a drug for treating and/or preventing male infertility. The extract comprises alkaloids with a weight content of 3% or more, and/or polysaccharides with a weight content of not more than 70%, and/or flavonoids with a weight content of not more than 10%, and/or an amino acid with a weight content of not more than 50%, and/or other components. The extract can be used for male infertility related to male sexual dysfunction and male reproductive dysfunction.

## Description

### TECHNICAL FIELD

The present disclosure relates to the field of biological medicine and specifically to use of a mulberry extract in the preparation of a drug for treating and/or preventing male infertility.

### BACKGROUND

Where a couple of childbearing age fails to get natural pregnancy after more than one year of regular sexual intercourse without taking any contraceptive measures, this condition attributable to the male partner is referred to as male infertility. It is estimated that 15% of couples of childbearing age worldwide experience fertility problems, of which the male factors account for approximately 50%. Male infertility is not a distinct disease but rather the consequence of multiple causes and contributing factors. The main risk factors include age, unhealthy habits (smoking and alcohol drinking), obesity, poor lifestyle habits (staying up late and lack of exercise), pathogen infections, varicocele, adverse psychological and emotional factors, etc.

Clinically, male infertility is classified into two categories: sexual dysfunction and normal sexual function. The latter is often referred to as "reproductive dysfunction". In clinical practice, sexual dysfunction and reproductive dysfunction interact with and promote each other, and they often do not exist independently. Sexual dysfunction clinically manifests as libido disorders, erectile dysfunction, ejaculation disorders, and lack of orgasm. Libido disorders are mainly associated with the reduced testosterone levels or altered receptor function; erectile dysfunction is mainly associated with damage to corpus cavernosum smooth muscle or dysfunction of endothelial cells; ejaculation dysfunction is mainly associated with smooth muscle injury, autonomic nerve injury, and reduced testosterone levels; and lack of orgasm is mainly associated with androgen and pudendal nerve sensory disorders. Among them, erectile dysfunction and ejaculation disorders severely affect the union of sperm and fertilized egg and are thus closely bound up with male infertility. Reproductive dysfunction is mostly caused by abnormal sperm parameters, such as reduced sperm count and poor sperm motility. Its clinical manifestations often include vas deferens obstruction, abnormal sperm quality, semen abnormality, varicocele, inflammation, and the like. Of these, the sperm quality is a key indicator for evaluating male infertility. Based on semen analysis results, male infertility may be further classified into azoospermia, oligozoospermia, asthenozoospermia, necrospermia, and normozoospermic infertility.

Male reproductive dysfunction and sexual dysfunction severely affect people's quality of life and fertility. However, existing therapeutic drugs often fall short of expectations, offering unsatisfactory clinical efficacy. Particularly in the context of an increasingly aging population and the national two- and three-child policies, developing safe and effective drugs that can improve sperm quality and motility while ameliorating sexual dysfunction is not only of significant scientific and medical value but also of great social value.

### SUMMARY

An objective of the present disclosure is to provide use of a mulberry extract in the preparation of a drug for treatment and/or prevention of male infertility. In another aspect, provided is use of a mulberry extract for treatment and/or prevention of male infertility. In another aspect, provided is a method for treating male infertility, comprising administering a therapeutically or prophylactically amount of mulberry extract to a subject in need thereof. The mulberry extract has a significant therapeutic effect on male infertility associated with sexual dysfunction and reproductive dysfunction.

In another aspect, provided is use of a mulberry extract in the preparation of a product for *in vitro* inhibition of high glucose-induced apoptosis of testicular Leydig cells. In another aspect, provided is use of a mulberry extract for *in vitro* inhibition high glucose-induced apoptosis of testicular Leydig cells. In another aspect, provided is a method for inhibiting high glucose-induced apoptosis of testicular Leydig cells *in vitro*, comprising administering a therapeutically or prophylactically amount of mulberry extract to a subject in need thereof.

In another aspect, provided is use of a mulberry extract in the preparation of a product for *in vitro* amelioration of high glucose-induced endothelial cell injury. In another aspect, provided is use of a mulberry extract for *in vitro* amelioration of high glucose-induced endothelial cell injury. In another aspect, provided is a method for ameliorating high glucose-induced endothelial cell injury *in vitro*, comprising administering a therapeutically or prophylactically amount of mulberry extract to a subject in need thereof.

In the present disclosure, based on the weight percentage of the sum of components of the mulberry extract as 100%, the mulberry extract comprises alkaloids in an amount of 3% by weight or more (optionally comprises alkaloids in an amount of 3 to 99% by weight, further optionally comprises alkaloids in an amount of 15 to 99% by weight, further optionally comprises alkaloids in an amount of 30 to 99% by weight, further optionally comprises alkaloids in an amount of 40 to 99% by weight, further optionally comprises alkaloids in an amount of 50 to 99% by weight, further optionally comprises alkaloids in an amount of 60 to 99% by weight, further optionally comprises alkaloids in an amount of 35 to 70% by weight, or further optionally comprises alkaloids in an amount of 60 to 75% by weight); and/or
comprises polysaccharides in an amount of not more than 70% by weight (optionally comprises polysaccharides in an amount of 0.2 to 70% by weight, optionally further comprises polysaccharides in an amount of 0.2 to 50% by weight, optionally further comprises polysaccharides in an amount of 0.2 to 35% by weight, optionally further comprises polysaccharides in an amount of 0.2 to 25% by weight, optionally further comprises polysaccharides in an amount of 0.2 to 23% by weight, or optionally further comprises polysaccharides in an amount of 20 to 25% by weight), and/or
comprises flavonoids in an amount of not more than 10% by weight (optionally comprises flavonoids in an amount of 0.05 to 5% by weight, optionally further comprises flavonoids in an amount of 0 to 2% by weight, optionally further comprises flavonoids in an amount of 0.05 to 2% by weight, optionally further comprises flavonoids in an amount of 0.5 to 1.5% by weight, optionally further comprises flavonoids in an amount of 0 to 1% by weight, or optionally further comprises flavonoids in an amount of 0.05 to 1% by weight), and/or
comprises amino acids in an amount of not more than 50% by weight (optionally comprises amino acids in an amount of 0 to 30% by weight, optionally further comprises amino acids in an amount of 0 to 25% by weight, optionally further comprises amino acids in an amount of 0 to 20% by weight, optionally further comprises amino acids in an amount of 0 to 5% by weight, optionally further comprises amino acids in an amount of 3 to 25% by weight, or optionally further comprises amino acids in an amount of 5 to 20% by weight), and/or
an additional component (optionally in an amount of 0 to 25% by weight, further optionally in an amount of 0 to 20% by weight, optionally in an amount of 0 to 15% by weight, optionally in an amount of 0 to 11% by weight, optionally in an amount of 2 to 20% by weight, or optionally in an amount of 4 to 8% by weight).

Preferably, the male infertility is a male sexual dysfunction and/or a male reproductive dysfunction.

Preferably, the male sexual dysfunction and/or the male reproductive dysfunction are caused by an endocrine abnormality and/or a metabolic abnormality; preferably, the metabolic abnormality is a glucolipid metabolic abnormality; preferably, the endocrine abnormality is a hormone level abnormality.

Preferably, the glucolipid metabolic abnormality is at least one selected from diabetes, hyperglycemia, overweight, obesity, and hyperlipidemia; preferably, the hormone level abnormality is a sex hormone level abnormality, preferably an androgen level abnormality.

Preferably, the male sexual dysfunction is at least one selected from libido disorders, erectile dysfunction, ejaculation disorders, and lack of orgasm; preferably, the male sexual dysfunction is erectile dysfunction and/or ejaculation disorders.

Preferably, the male reproductive dysfunction is at least one selected from vas deferens obstruction, abnormal sperm quality, semen abnormality, varicocele, and inflammation; further preferably, the abnormal sperm quality is at least one selected from azoospermia, oligozoospermia, asthenozoospermia, and necrospermia. Preferably, the male reproductive dysfunction is oligozoospermia, asthenozoospermia and/or oligoasthenozoospermia.

Preferably, the treatment and/or prevention of the male infertility specifically manifest in at least one of:
1) promoting spermatogenesis;
2) enhancing sperm motility;
3) improving sperm quality;
4) improving testicular index;
5) improving a structure of seminiferous tubules and a morphology and count of spermatogenic cells in testicular tissue;
6) improving a level of a relevant sex hormone; preferably, the sex hormone is androgen; further preferably, the androgen is testosterone (T);
7) improving an expression level of an androgen synthase; the androgen synthase comprises at least one of a steroidogenic acute regulatory protein (STAR), which is a rate-limiting enzyme for testosterone synthesis; and a cytochrome P450 17α-hydroxylase (CYP17A1) and a 17β-hydroxysteroid dehydrogenase (17β-HSD), which are important enzymes for testosterone synthesis;
8) improving an expression level of an androgen receptor; and
9) improving penile tissue injury and/or cell injury; preferably, the penile cells are endothelial cells of penile corpora cavernosa; preferably, improving a NO content in the endothelial cells of the penile corpora cavernosa; preferably, the improvement in the penile tissue injury specifically comprises an improvement in at least one of an average thickness of cavernous scaffolds, a connectivity density, a degree of separation, and a mean intercept length of sinusoidal spaces.

Further preferably, the treatment and/or prevention of the male reproductive dysfunction specifically manifest in at least one of:
1) promoting spermatogenesis;
2) enhancing sperm motility;
3) improving sperm quality;
4) improving testicular index;
5) improving a structure of seminiferous tubules and a morphology and count of spermatogenic cells in testicular tissue; preferably, the spermatogenic cells comprise at least one of spermatogonocytes, primary spermatocytes, secondary spermatocytes, and spermatoblasts;
6) improving a level of a relevant sex hormone; preferably, the sex hormone is androgen; further preferably, the androgen is testosterone (T);
7) improving an expression level of an androgen synthase; preferably, the androgen synthase comprises at least one of a steroidogenic acute regulatory protein STAR, which is a rate-limiting enzyme for testosterone synthesis; and a cytochrome P450 17α-hydroxylase (CYP17A1) and a 17β-hydroxysteroid dehydrogenase 17β-HSD, which are important enzymes for testosterone synthesis;
8) improving an expression level of an androgen receptor; and
9) improving testicular cell injury; preferably, the testicular cells are testicular Leydig cells;
further preferably, the treatment and/or prevention of the male sexual dysfunction specifically manifest in at least one of:
1) improving a level of a relevant sex hormone; preferably, the sex hormone is androgen; further preferably, the androgen is testosterone (T);
2) improving an expression level of an androgen synthase; preferably, the androgen synthase comprises at least one of a steroidogenic acute regulatory protein STAR, which is a rate-limiting enzyme for testosterone synthesis; and a cytochrome P450 17α-hydroxylase (CYP17A1) and a 17β-hydroxysteroid dehydrogenase 17β-HSD, which are important enzymes for testosterone synthesis;
3) improving an expression level of an androgen receptor;
4) improving penile tissue and/or cell injury; preferably, the penile cells are endothelial cells of penile corpora cavernosa; preferably, improving a NO content in the endothelial cells of the penile corpora cavernosa; preferably, the improvement in the penile tissue injury specifically comprises an improvement in at least one of an average thickness of cavernous scaffolds, a connectivity density, a degree of separation, and a mean intercept length of sinusoidal spaces; and
5) improving an IIEF-15 score; preferably, the IIEF-15 score includes at least one of erectile function, satisfaction with penetration, orgasm, libido, and overall satisfaction.

In one optional embodiment of the present disclosure, based on the weight percentage of the sum of components of the mulberry extract as 100%, the weight contents of the components in the mulberry extract are as follows:

| | |
|---|---|
| alkaloids | 3-99%, |
| polysaccharides | 0.2-70%, |
| flavonoids | 0-10%, |
| amino acids | 0-50%, |
| additional component | 0-25%; |

optionally, based on the weight percentage of the sum of components of the mulberry extract as 100%, the weight contents of the components in the mulberry extract are as follows:

| | |
|---|---|
| alkaloids | 30-99%, |
| polysaccharides | 0.2-35%, |
| flavonoids | 0-2%, |
| amino acids | 0-30%, |
| additional component | 0-20%; |

optionally, based on the weight percentage of the sum of components of the mulberry extract as 100%, the weight contents of the components in the mulberry extract are as follows:

| | |
|---|---|
| alkaloids | 30-99%, |
| polysaccharides | 0.2-35%, |
| flavonoids | 0.05-2%, |
| amino acids | 0-25%, |
| additional component | 0-20%; |

optionally, based on the weight percentage of the sum of components of the mulberry extract as 100%, the weight contents of the components in the mulberry extract are as follows:

| | |
|---|---|
| alkaloids | 40-99%, |
| polysaccharides | 0.2-25%, |
| flavonoids | 0.05-1%, |
| amino acids | 0-25%, |
| additional component | 0-15%; |

optionally, based on the weight percentage of the sum of components of the mulberry extract as 100%, the weight contents of the components in the mulberry extract are as follows:

| | |
|---|---|
| alkaloids | 50-99%, |
| polysaccharides | 0.2-23%, |
| flavonoids | 0-1%, |
| amino acids | 0-20%, |
| additional component | 0-11%; |

optionally, based on the weight percentage of the sum of components of the mulberry extract as 100%, the weight contents of the components in the mulberry extract are as follows:

| | |
|---|---|
| alkaloids | 50-99%, |
| polysaccharides | 0.2-35%, |
| flavonoids | 0-2%, |
| amino acids | 0-30%, |
| additional component | 0-20%; |

optionally, based on the weight percentage of the sum of components of the mulberry extract as 100%, the weight contents of the components in the mulberry extract are as follows:

| | |
|---|---|
| alkaloids | 60-99%, |
| polysaccharides | 0.2-23%, |
| flavonoids | 0-1%, |
| amino acids | 0-5%, |
| additional component | 0-11%; |

further optionally, based on the weight percentage of the sum of components of the mulberry extract as 100%, the weight contents of the components in the mulberry extract are as follows:

| | |
|---|---|
| alkaloids | 50-65%, |
| polysaccharides | 20-25%, |
| flavonoids | 0.5-1.5%, |
| amino acids | 3-25%, |
| additional component | 2-20%; |

further optionally, based on the weight percentage of the sum of components of the mulberry extract as 100%, the weight contents of the components in the mulberry extract are as follows:

| | |
|---|---|
| alkaloids | 50-65%, |
| polysaccharides | 20-25%, |
| flavonoids | 0.5-1.5%, |
| amino acids | 5-20%, |
| additional component | 4-8%; |

further optionally, based on the weight percentage of the sum of components of the mulberry extract as 100%, the weight contents of the components in the mulberry extract are as follows:
alkaloids 40-65%,
polysaccharides 22-25%,
flavonoids 0.5-1%,
amino acids 5-25%,
additional component 5-10%,
more optionally, based on the mulberry extract, the weight content of each component is as follows:

| | |
|---|---|
| alkaloids | 30-90%, |
| polysaccharides | 0.2-35%, |
| flavonoids | 0-2%, |
| amino acids | 0-30%, |
| additional component | 0-20%; |

optionally, based on the mulberry extract, the weight content of each component is as follows:

| | |
|---|---|
| alkaloids | 30-90%, |
| polysaccharides | 0.2-35%, |
| flavonoids | 0.05-2%, |
| amino acids | 0-25%, |
| additional component | 0-20%; |

optionally, based on the mulberry extract, the weight content of each component is as follows:

| | |
|---|---|
| alkaloids | 30-80%, |
| polysaccharides | 0.2-35%, |
| flavonoids | 0-2%, |
| amino acids | 0-30%, |
| additional component | 0-11%; |

optionally, based on the mulberry extract, the weight content of each component is as follows:

| | |
|---|---|
| alkaloids | 30-80%, |
| polysaccharides | 5-35%, |
| flavonoids | 0.1-2%, |
| amino acids | 4-25%, |
| additional component | 8-11%; |

optionally, based on the mulberry extract, the weight content of each component is as follows:

| | |
|---|---|
| alkaloids | 30-70%, |
| polysaccharides | 0.2-35%, |
| flavonoids | 0-2%, |
| amino acids | 0-25%, |
| additional component | 0-8%; |

optionally, based on the mulberry extract, the weight content of each component is as follows:

| | |
|---|---|
| alkaloids | 30-70%, |
| polysaccharides | 20-35%, |
| flavonoids | 0.6-2%, |
| amino acids | 5-25%, |
| additional component | 4-8%; |

optionally, based on the mulberry extract, the weight content of each component is as follows:

| | |
|---|---|
| alkaloids | 35-70%, |
| polysaccharides | 0.2-35%, |
| flavonoids | 0-2%, |
| amino acids | 0-25%, |
| additional component | 0-8%; |

optionally, based on the mulberry extract, the weight content of each component is as follows:

| | |
|---|---|
| alkaloids | 35-70%, |
| polysaccharides | 20-35%, |
| flavonoids | 0.6-2%, |
| amino acids | 5-25%, |
| additional component | 4-8%; |

optionally, based on the mulberry extract, the weight content of each component is as follows:

| | |
|---|---|
| alkaloids | 40-70%, |
| polysaccharides | 0.2-25%, |
| flavonoids | 0.05-1%, |
| amino acids | 0-25%, |
| additional component | 0-10%; |

optionally, based on the mulberry extract, the weight content of each component is as follows:

| | |
|---|---|
| alkaloids | 40-70%, |
| polysaccharides | 20-25%, |
| flavonoids | 0.05-1%, |
| amino acids | 5-25%, |
| additional componnent | 0-10%; |

optionally, based on the mulberry extract, the weight content of each component is as follows:

| | |
|---|---|
| alkaloids | 30-65%, |
| polysaccharides | 0.2-35%, |
| flavonoids | 0-2%, |
| amino acids | 0-25%, |
| additional component | 0-8%; |

optionally, based on the mulberry extract, the weight content of each component is as follows:

| | |
|---|---|
| alkaloids | 30-65%, |
| polysaccharides | 20-35%, |
| flavonoids | 0.6-2%, |
| amino acids | 5-25%, |
| additional componnent | 4-8%; |

optionally, based on the mulberry extract, the weight content of each component is as follows:

| | |
|---|---|
| alkaloids | 15-99% |
| polysaccharides | 0.2-20%, |
| flavonoids | 0-10%, |
| amino acids | 0-45%, |
| additional componnent | 0-15%; |

optionally, based on the mulberry extract, the weight content of each component is as follows:

| | |
|---|---|
| alkaloids 1 | 5-90% |
| polysaccharides | 3-20%, |
| flavonoids | 0-10%, |
| amino acids | 0-45%, |
| additional component | 0-15%; |

optionally, based on the mulberry extract, the weight content of each component is as follows:

| | |
|---|---|
| alkaloids 1 | 5-80% |
| polysaccharides | 5-35%, |
| flavonoids | 0-10%, |
| amino acids | 0-45%, |
| additional componnent | 0-15%; |

optionally, based on the mulberry extract, the weight content of each component is as follows:

| | |
|---|---|
| alkaloids | 15-70% |
| polysaccharides | 0.2-20%, |
| flavonoids | 0-10%, |
| amino acids | 0-45%, |
| additional component | 0-15%; |

optionally, based on the mulberry extract, the weight content of each component is as follows:

| | |
|---|---|
| alkaloids | 15-70% |
| polysaccharides | 0.2-20%, |
| flavonoids | 0-10%, |
| amino acids | 5-45%, |
| additional comp onent | 0-15%; |

optionally, based on the mulberry extract, the weight content of each component is as follows:

| | |
|---|---|
| alkaloids | 15-65% |
| polysaccharides | 0.2-20%, |
| flavonoids | 0-10%, |
| amino acids | 0-45%, |
| additional compo onent | 0-15%; |

optionally, based on the mulberry extract, the weight content of each component is as follows:

| | |
|---|---|
| alkaloids | 15-65% |
| polysaccharides | 0.2-20%, |
| flavonoids | 0-10%, |
| amino acids | 5-45%, |
| additional comp onent | 0-15%; |

in the mulberry extract, the weight content of each component is as follows:

| | |
|---|---|
| alkaloids | 15-30%, |
| polysaccharides | 0.2-40%, |
| flavonoids | 0-10%, |
| amino acids | 20-50%, |
| additional component | 0-15%; |

in the mulberry extract, the weight content of each component is as follows:

| | |
|---|---|
| alkaloids | 3-70%, |
| polysaccharides | 0.2-70%, |
| flavonoids | 0-10%, |
| amino acids | 0-10%, |
| additional component | 0-10%; |

in the mulberry extract, the weight content of each component is as follows:

| | |
|---|---|
| alkaloids | 3-70%, |
| polysaccharides | 20-70%, |
| flavonoids | 0-10%, |
| amino acids | 5-10%, |
| additional component | 0-10%; |

in the mulberry extract, the weight content of each component is as follows:

| | |
|---|---|
| alkaloids | 3-30%, |
| polysaccharides | 35-70%, |
| flavonoids | 0-10%, |
| amino acids | 0-25%, |
| additional component | 0-10%; |

in the mulberry extract, the weight content of each component is as follows:

| | |
|---|---|
| alkaloids | 3-30%, |
| polysaccharides | 35-70%, |
| flavonoids | 0-10%, |
| amino acids | 0-25%, |
| additional component | 0-10%; |

in the mulberry extract, the weight content of each component is as follows:

| | |
|---|---|
| alkaloids | 3-40%, |
| polysaccharides | 25-70%, |
| flavonoids | 0-10%, |
| amino acids | 0-25%, |
| additional component | 0-10%; |

in the mulberry extract, the weight content of each component is as follows:

| | |
|---|---|
| alkaloids | 3-40%, |
| polysaccharides | 25-70%, |
| flavonoids | 0-10%, |
| amino acids | 10-25%, |
| additional component | 0-10%; |

in the mulberry extract, the weight content of each component is as follows:

| | |
|---|---|
| alkaloids | 3-50%, |
| polysaccharides | 20-70%, |
| flavonoids | 0-10%, |
| amino acids | 0-20%, |
| additional component | 0-10%; |

in the mulberry extract, the weight content of each component is as follows:

| | |
|---|---|
| alkaloids | 3-50%, |
| polysaccharides | 20-70%, |
| flavonoids | 0-10%, |
| amino acids | 10-20%, |
| additional component | 0-10%; |

in the mulberry extract, the weight content of each component is as follows:

| | |
|---|---|
| alkaloids | 3-65%, |
| polysaccharides | 20-70%, |
| flavonoids | 0-10%, |
| amino acids | 0-10%, |
| additional component | 0-10%; |

in the mulberry extract, the weight content of each component is as follows:

| | |
|---|---|
| alkaloids | 3-65%, |
| polysaccharides | 20-70%, |
| flavonoids | 0-10%, |
| amino acids | 5-10%, |
| additional component | 0-10%; |

in the mulberry extract, the weight content of each component is as follows:

| | |
|---|---|
| alkaloids | 80-99%, |
| polysaccharides | 0.2-10%, |
| flavonoids | 0-1%, |
| amino acids | 0-5%, |
| additional component | 0-15%, |

the weight content of each component may be a combination of any numerical values described above.

Optionally, the alkaloids comprise one or more of 1-deoxynojirimycin (DNJ), N-methly-1-deoxynojirimycin, fagomine (FAG), 3-epi-fagomine, 1,4-dideoxy-1,4-imino-D-arabinitol (DAB), calystegin B2, calystegin C1, 2-O-(α-D-galactopyranosyl)-1-deoxynojirimycin, 6-O-(β-D-glucopyranosyl)-1-deoxynojirimycin, and 1,4-dideoxy-1,4-imino-(2-O-β-D-glucopyranosyl)-D-arabinitol.

Optionally, a weight percentage of DNJ is not less than 50% (optionally 60 to 99%) of total alkaloids.

Optionally, the heavy metal content of the mulberry extract is not more than 10 ppm.

The drug is an oral dosage form; optionally, the drug is a tablet, a capsule, an oral solution, an oral emulsion, a pill, a granule, a syrup, and a powder.

In the present disclosure, the mulberry extract may also be provided in the form of a commercially available mulberry twig alkaloids tablets (SFDA approval No.: Z20200002).

Optionally, the mulberry extract may be prepared by the method described in CN 110393738A. In one embodiment, the preparation of the mulberry extract comprises the following steps: preparing a crude extraction solution of a plant of Moraceae; optionally, separating via a cation resin and/or an anion resin; optionally, subjecting effluent obtained from resin separation to alcohol precipitation, and collecting a supernatant; and optionally, subjecting the supernatant to concentration and/or drying.

Optionally, in the present disclosure, the preparation of the mulberry extract comprises the following steps: 1) preparing a crude extraction solution of a plant of Moraceae; 2) separating the crude extraction solution via a cation resin and/or an optional anion resin to obtain effluent from resin separation; optionally, step 3): subjecting the effluent obtained from resin separation in step 2) to alcohol precipitation, and collecting a supernatant; and 4) subjecting the supernatant to concentration and/or drying. Optionally, the effluent obtained from resin separation in step 2) is subjected to concentration and/or drying prior to alcohol precipitation.

Optionally, the plant of Moraceae is one or a combination of more of *Morus multicaulis* Perrott., *Morus alba* L., *Morus atropurpurea* Roxb, *Morusmizuho* Hotta, *Morus wittiorum* Hand Mazz., *Morus laevigata* Wall, *Morus nigra* Linn., *Morus cathayana* Hemsi., *Morus serrata* Roxb., *Morus mongolica* Schneid., *Morus bombycis* Koidz., *Morus notabilis* Schneid., *Morus nigriformis* Koidz., *Morusyunnanensis* Koidz., *Morus australis* Poir., *Morus mongolica* (Bur.) Schneid var. diabolica Koidz., *Morus alba* L. var. macrophylla loud, *Morus alba* Var. Pendula Dippel, Morus alba L. var. venosa Delili, a mulberry variety bred from the above mulberry species, and a hybrid mulberry obtained from selective intra-species or inter-species breeding of the above mulberry species;
optionally, the plant of Moraceae is one or more of *Morus atropurpurea* Roxb, *Morus multicaulis* Perrott., *Morus alba* L., *Morus serrata* Roxb., *Morus bombycis* Koidz. or hybrid mulberry, and the hybrid mulberry is optionally Yuesang No.11, Guisangyou No.62 or Sangteyou No.2. Various parts such as the leaf, root, branch, bark, bud, stem, and fruit of the plant may be used. Ramulus Mori, Folium Mori or Cortex Mori may be used.

In the present disclosure, the mulberry extract is optionally a Ramulus Mori extract, a Cortex Mori extract, a Folium Mori extract, or a mixed extract thereof.

In one embodiment, the mulberry extract is prepared by the following steps: the plant of Moraceae is crushed, and extracted by heating reflux with water and/or alcoholic solution or acid water in a solvent amount of 3 to 20 times that of the raw medicinal materials; the extraction is repeated for 1 to 3 times; the extracting solutions are combined, concentrated, loaded onto a cation exchange resin, and then eluted with 0.2 to 3N of ammonia water; the eluate is loaded onto an anion exchange resin; the unadsorbed parts are collected, added with ethanol, precipitated to remove impurities, concentrated and/or dried to afford the extract.

In one embodiment, the mulberry extract is prepared by the following steps: the plant of Moraceae is crushed, and extracted by heating reflux with water and/or alcoholic solution or acid water in a solvent amount of 3 to 20 times that of the raw medicinal materials; the extraction is repeated for 1 to 3 times; the extracting solutions are combined, concentrated, loaded onto a cation exchange resin, and then eluted with 0.2 to 3N of ammonia water; and the eluate is loaded onto an anion exchange resin; the unadsorbed parts are collected, concentrated and/or dried to afford the extract.

In one embodiment, the mulberry extract is prepared by the following steps: the plant of Moraceae is crushed, and extracted by heating reflux with water and/or alcoholic solution or acid water in a solvent amount of 3 to 20 times that of the raw medicinal materials; the extraction is repeated for 1 to 3 times; the extracting solutions are combined, concentrated, loaded onto a cation exchange resin, and then eluted with 0.2 to 3N of ammonia water; and the eluate is concentrated and/or dried to afford the extract.

In one embodiment, the mulberry extract is prepared by the following steps: the plant of Moraceae is crushed, and extracted by heating reflux with water in a solvent amount of 3 to 20 times (optionally 4 to 15 times, or further optionally 4 to 12 times) that of the raw medicinal materials; the extraction is repeated for 1 to 3 times (the extraction time is optionally 0.5 to 3 h each time, or further optionally 1 to 2 h each time); the extracting solutions are combined, concentrated, loaded onto a cation exchange resin, and then eluted with 0.2 to 3N of ammonia water; the eluate is loaded onto an anion exchange resin; and the unadsorbed parts are collected (i.e., anion resin effluent), added with ethanol, precipitated to remove impurities, concentrated and/or dried to afford the extract.

Optionally, the concentrated crude extraction solution may also be subjected to alcohol precipitation prior to the resin separation in step 2). During the alcohol precipitation, ethanol is added to the crude extraction solution, the mixture is stirred and mixed evenly, the stirring is stopped, and the resultant is allowed to stand for a certain period of time to precipitate the insoluble matters therein. Optionally, in terms of L/kg, the volume-to-mass ratio of ethanol added to the plant raw material is 0.2 to 20 times or optionally 0.4 to 10 times. Further optionally, alcohol precipitation is conducted by using an alcohol precipitation tank. Optionally, the stirring speed during alcohol precipitation is 10 to 600 rpm, optionally 40 to 500 rpm, or further optionally 80 to 400 rpm or 300 rpm.

Optionally, after being packed into the column, the cation resin is subjected to activation by being washed with an acidic solution, an alkaline solution and an acidic solution successively. Optionally, the resin is washed with an alkaline solution until the pH of the eluate is 8.0 to 9.5, preferably 8.5 to 9.5; optionally, the alkaline solution is ammonia solution, sodium hydroxide solution, potassium hydroxide solution or sodium carbonate solution; optionally, the concentration of the alkaline solution is 0.5 to 4 mol/L or optionally 1 to 2 mol/L. Optionally, the resin is washed with an acidic solution until the pH of the eluate is 3.0 to 7.0 or optionally 4.5 to 6.5. Optionally, the acidic solution is selected from hydrochloric acid solution, phosphoric acid solution and disodium hydrogen phosphate-citric acid buffer, and optionally, the concentration of the acidic solution is 0.5 to 4 mol/L, or optionally 1.5 to 2 mol/L. Optionally, the cation resin may also be washed with deionized water having a volume of 3 to 5 times the column volume after the last washing with the acidic solution.

Optionally, the cation resin is one or a combination of more of 732-type strongly acidic styrene-based cation exchange resin, 002SC-type strongly acidic styrene-based cation exchange resin, 734-type strongly acidic styrene-based cation exchange resin, D001-type macroporous and strongly acidic styrene-based cation exchange resin, D113-type macroporous and weakly acidic cation resin, and D254-type macroporous and strongly alkaline quaternary ammonium-type cation exchange resin.

Optionally, the weight ratio of the cation resin to the charged plant raw material is 1:1 to 1:30 (optionally, 1:1 to 1:25, 1:2 to 1:20, 1:2 to 1:15, 1:2 to 1:10, 1:2 to 1:7, 1:2 to 1:3).

After loading the crude extraction solution of the plant onto the cation resin, the loaded cation resin is subjected to elution with an eluent. Optionally, the eluent is a salt solution or an alkaline solution containing cations, optionally one or more of sodium chloride, ammonium chloride, ammonium sulfate, ammonium nitrate, ammonia water, potassium chloride, and sodium hydroxide.

Optionally, the eluent has a concentration of 0.04 to 5 mol/L (optionally 0.5 to 2.5 mol/L, 0.2 to 3 mol/L, or further optionally 0.5 to 2.5 mol/L).

Optionally, the flow rate of the eluent is 1 to 15 BV/h (optionally 5 to 10 BV/h, or further optionally 5 to 6 BV/h).

Optionally, the weight of the eluent used for the separation via cation resin is 0.1 to 30 times the weight of the charged plant raw material. Optionally, the elution is conducted with the eluent, of which the weight is 0.5 to 10 times the weight of the charged plant raw material.

The starting point of the collection may be determined according to the pH of the effluent obtained from the cation resin, for example, when an alkaline solution such as ammonia water is used for conducting elution, the eluate is collected when the pH of the effluent obtained from the cation resin is higher than 7, or the starting point of the collection of the effluent is determined by utilizing a chromogenic reaction or a precipitation reaction; optionally, the collection is terminated when the volume of the collection solution reaches 0.1 to 10 times (further optionally, 0.2 to 5 times) the weight of the charged plant raw material, and the collection solution is optionally purified over the anion resin column.

Upon purification over the anion resin column, optionally, the anion resin is subjected to activation by being washed with an alkaline solution, an acidic solution and an alkaline solution successively after being packed into the column.

Optionally, the resin is washed with an alkaline solution until the pH of the eluate is 8.0 to 9.5 or optionally 8.5 to 9.5;
optionally, the alkaline solution is ammonia solution, sodium hydroxide solution, potassium hydroxide solution or sodium carbonate solution; optionally, the concentration of the alkaline solution is 0.5 to 4 mol/L or optionally 1 to 2 mol/L.

Optionally, the resin is washed with an acidic solution until the pH of the eluate is 3.0 to 7.0 or optionally 4.5 to 6.5. Optionally, the acidic solution is selected from hydrochloric acid solution, phosphoric acid solution and disodium hydrogen phosphate-citric acid buffer, and optionally, the concentration of the acidic solution is 0.5 to 4 mol/L, or optionally 1 to 2 mol/L.

Optionally, the anion resin is one or a combination of more of 717-type strongly alkaline styrene-based anion resin, 717-type strongly alkaline styrene-based anion exchange resin, D201-type macroporous and strongly alkaline styrene-based anion exchange resin, D218-type macroporous and strongly alkaline acrylic-based anion exchange resin, D301-G-type macroporous and weakly acidic styrene-based anion exchange resin, and D301-type macroporous and weakly alkaline styrene-based anion exchange resin.

Optionally, the amount of the anion resin used and the charged plant raw material has a weight ratio ranging from 1:1 to 1:80 (optionally, 1:1 to 1:64, 1:1 to 1:32, 1:1 to 1:24, 1:5 to 1:16, 1:3).

Collection begins when the liquid flows out of the anion resin. Optionally, the collection is terminated when the volume of the collection solution reaches 0.05 to 10 times (optionally, 0.1 to 5 times) the weight of the charged plant raw material.

Optionally, the ethanol used for alcohol precipitation and the charged plant raw material has a weight ratio ranging from 1:4 to 1:600 (optionally, 1:20 to 1:300, further optionally 1: 20 to 1:50, 1:40, 1:80, 1:22). In the alcohol precipitation, the stirring speed is 10 to 600 rpm (optionally 40 to 500 rpm, or 80 to 400 rpm). The time of alcohol precipitation is 12 to 24 h.

Furthermore, the step of subjecting the effluent obtained from anion resin separation to centrifugation to remove impurities or filtration through a micro-filtration membrane to remove impurities and then to concentration through the reverse osmosis membrane is further included prior to the alcohol precipitation. The specific gravity of the concentrated liquid may be 1.0 to 1.3, or optionally 1.1 to 1.25.

Optionally, the drug further comprises a pharmaceutically acceptable carrier. The carrier is an inactive component that conforms to the administration route or the mode of administration and has no toxic effect on the human body. The carrier may be a solid or liquid excipient. Solid excipients, for example, include microcrystalline cellulose, mannitol, lactose, pregelatinized starch, low-substituted hydroxypropyl cellulose, crospovidone, sodium carboxymethyl starch, aspartame, calcium hydrogen phosphate, sodium lactate, poloxamer, sodium dodecyl sulfate, sodium carboxymethyl cellulose, gelatin, xanthan gum, povidone, starch, magnesium stearate, sodium carboxymethyl starch, and talc. Liquid excipients, for example, include water, ethanol, syrup, and glycerin.

In the present disclosure, the mulberry extract is simply referred to as SZ-A.

### Advantageous Effects of the Invention:

The mulberry extract can ameliorate infertility, including male sexual dysfunction and male reproductive disorders. The mulberry extract can increase the total sperm count, motile sperm count, and sperm motility, improve penile tissue injury, significantly increase the testosterone levels in testicular tissue, significantly increase the expression of STAR as a rate-limiting enzyme for testosterone synthesis, CYP17A1 as an important enzyme for testosterone synthesis, and the androgen receptor AR, significantly improve the structure of testicular seminiferous tubules and the status of Leydig cells, alleviate inflammatory cell infiltration, and increase the number of layers and the number of spermatogenic cells at all stages, which appear tightly connected and neatly arranged.

### BRIEF DESCRIPTION OF THE DRAWINGS

FIG. 1 shows the results of (A) total sperm count and (B) motile sperm count of male SD rats in each test group of Test Example 1.
FIG. 2 shows the results of sperm count, motile sperm count, and sperm motility in GAN-induced male mice in each test group of Test Example 2 (n=5, data are presented as "mean ± standard error"; the difference between groups is compared using one-way analysis of variance; compared with the Model group, * P < 0.05, **P < 0.01);
FIG. 3 shows the testosterone levels in testicular tissue of GAN-induced male mice in each test group of Test Example 2 (n=10, data are presented as "mean ± standard error"; the difference between groups is compared using one-way analysis of variance; compared with the Model group, *P < 0.05, **P < 0.01);
FIG. 4 shows the STAR levels in testicular tissue of GAN-induced male mice in each test group of Test Example 2 (n=3, data are presented as "mean ± standard error"; the difference between groups is compared using one-way analysis of variance; compared with the Model group, *P < 0.05, **P < 0.01);
FIG. 5 shows the CYP17A1 levels in testicular tissue of GAN-induced male mice in each test group of Test Example 2 (n=3, data are presented as "mean ± standard error"; the difference between groups is compared using one-way analysis of variance; compared with the Model group, *P < 0.05, **P < 0.01);
FIG. 6 shows the AR levels in testicular tissue of GAN-induced male mice in each test group of Test Example 2 (n=3, data are presented as "mean ± standard error"; the difference between groups is compared using one-way analysis of variance; compared with the Model group, *P < 0.05, **P < 0.01);
FIG. 7 shows a diagram for the morphology of testicular tissue of GAN-induced male mice after HE staining in each test group of Test Example 2;
FIG. 8 shows the number of spermatogenic cells at all stages in testicular tissue of GAN-induced male mice in each test group of Test Example 2 (data are presented as "mean ± standard error"; the difference between groups is compared using one-way analysis of variance; compared with the Model group, *P < 0.05, **P < 0.01);
FIG. 9 shows the body weight results of HFD-induced obese C57BL/6J mice in each test group of Test Example 3;
FIG. 10 shows the results of the sperm count, motile sperm count, and sperm motility of HFD-induced obese C57BL/6J mice in each test group of Test Example 3; compared with the Model group, * P < 0.05, **P < 0.01;
FIG. 11 shows the testicular index results of HFD-induced obese C57BL/6J mice in each test group of Test Example 3; compared with the Model group, * P < 0.05, **P < 0.01;
FIG. 12 shows the corpus cavernosum structure of penile tissue stained with HE in HFD diet-induced obese C57BL/6J mouse model with reproductive dysfunction in Test Example 3; compared with the Model group, * P < 0.05, **P < 0.01, *** P<0.001;
FIG. 13 shows the results of the sperm count, motile sperm count, and sperm motility of mice in each test group of Test Example 4; compared with the Model group, * P < 0.05, **P < 0.01;
FIG. 14 shows the seminiferous tubule structure of testicular tissue stained with HE in the mice in each test group of Test Example 4;
FIG. 15 shows the number of spermatogenic cells at all stages and the total number of spermatogenic cells in mice of each test group in Test Example 4; compared with the Model group, * P < 0.05, **P < 0.01;
FIG. 16 shows the corpus cavernosum structure of penile tissue stained with HE in mice of each test group in Test Example 4; compared with the Model group, * P < 0.05, **P < 0.01, *** P<0.001;
FIG. 17 shows the effect of SZ-A on PA (palmitic acid)-induced testicular Leydig cell injury in Test Example 5;
FIG. 18 shows the effect 1 of SZ-A on high glucose-induced corpus cavernosum endothelial cell injury in Test Example 6; compared with the high glucose group, * P < 0.05, **P < 0.01, *** P<0.001;
FIG. 19 shows the effect 2 of SZ-A on high glucose-induced corpus cavernosum endothelial cell injury in Test Example 7 SZ-A; compared with the high glucose group, * P < 0.05, **P < 0.01, *** P<0.001;
FIG. 20 shows the results of mitochondria in endothelial cells observed by fluorescence microscopy in Test Example 8;
FIG. 21 shows the IIEF-15 score results of the clinical trial in Test Example 9.

### DETAILED DESCRIPTION

The present disclosure will be further illustrated by way of specific test examples below. It should be appreciated that the following experiments are intended only to illustrate the technical solution of the present disclosure and is not intended to limit the scope of protection of the present disclosure.

The mulberry extract according to the present disclosure can be prepared by the methods below.

### I. Preparative Examples of Mulberry Extract

### Preparative Example 1 of Mulberry Extract

1000 kg of fresh Ramulus Mori (*Morus serrata* Roxb. Yuesang No.11) were weighed and crushed, then added with 4000L of water, and extracted by heating reflux for 2 h. The extracting solutions were combined, and filtered to remove insoluble matters, thereby obtaining a crude extraction solution. The crude extraction solution was concentrated by heating until the solid content reached 4% by mass, held at 50°C, and served as a loading solution for the cation resin column.

150 kg of D113-type macroporous and weakly acidic phenylpropene-based cation resin was filled in the column, washed with 2 mol/L hydrochloric acid solution until the pH of the eluate was 4.5; washed with 1 mol/L sodium hydroxide solution until the pH of the eluate was 8.5; washed with 2 mol/L hydrochloric acid solution until the pH of the eluate was 4.5; and then rinsed with 5 times column volume of deionized water to complete the activation. The concentrated extracting solution was loaded, and then eluted with 1000L of 2.5 mol/L ammonia water at an elution speed of 6 BV/h. The eluate was collected when the effluent from the cation resin column was detected to be at pH > 7. When the collection solution was up to 900L, the collection was stopped. The collection solution was purified directly over the anion resin column.

62.5 kg of D218-type macroporous and strongly alkaline acrylic-based anion resin was filled in the column, washed with 1.5 mol/L sodium hydroxide solution until the pH of the eluate was 9.0; washed with 1.5 mol/L hydrochloric acid solution until the pH of the eluate was 3.5; and washed with 1.5 mol/L sodium hydroxide solution until the pH of the eluate was 9.0 to complete the activation. The eluate collected from the cation resin was loaded onto the anion resin. The effluent was collected and the collection was terminated when the effluent was up to 920L.

The collection solution was centrifuged to remove impurities, and then concentrated through the reverse osmosis membrane. The specific gravity of the concentrated liquid was 1.25. It was transferred to an alcohol precipitation tank, and 25L of anhydrous ethanol was added when the stirring paddle was at 500 rpm. After adding the ethanol, the solution was stopped stirring, and subjected to alcohol precipitation for 24 h. The supernatant was taken and concentrated under reduced pressure to obtain a Ramulus Mori extractum (i.e., mulberry extract SZ-A).

In the Ramulus Mori extractum, the alkaloids content was 52% by mass, the polysaccharides content was 22% by mass, the flavonoids content was 0.8% by mass, and the amino acids content was 20% by mass. In the alkaloids, the 1-DNJ content was 60% by mass, the FAG content was 17% by mass, and the DAB content was 15% by mass.

### Preparative Example 2 of Mulberry Extract

10 kg of fresh Ramulus Mori (Sangteyou No.2) was weighed and crushed, then added with 150L water in 2 times, and extracted by the decocting method for 3 h each time. The extracting solutions were combined, and filtered to remove the insoluble matters. The extracting solution was concentrated by heating until the solid content reached 8% by mass. It was transferred to an alcohol precipitation tank, and 2367.9 g of anhydrous ethanol (3L) was added when the stirring paddle was at 300 rpm. After adding the ethanol, the solution was stopped stirring, and subjected to alcohol precipitation for 24 h. The supernatant was taken as a loading solution for the cation resin column. 5 kg of 002SC-type strongly acidic styrene-based cation resin was filled in the column. The cation resin was activated according to the method described in Preparative Example 1. The extracting solution after concentration and alcohol precipitation was loaded, and then eluted with 100L of 5 mol/L potassium chloride at an elution speed of 5 BV/h. The effluent was detected with 20% silicotungstic acid, and started to collect when a white precipitate was generated. The collection was terminated when the collection solution reached 25L. The collection solution was purified directly over the anion resin column.

10 kg of 711-type strongly alkaline styrene-based anion resin was filled in the column. The anion resin was activated according to the method described in Example 1. The eluate collected from the cation resin was loaded onto the anion resin. The effluent was collected and the collection was terminated when the effluent was up to 15L. The collection solution was reloaded onto the cation resin, and separated twice via the cation resin and anion resin in sequence according to the methods described above.

The collection solution obtained after three column separation was centrifuged to remove impurities, and then concentrated through the reverse osmosis membrane. The specific gravity of the concentrated liquid was 1.25. It was transferred to an alcohol precipitation tank, and 125 g of anhydrous ethanol was added when the stirring paddle was at 1000 rpm. After adding the ethanol, the solution was stopped stirring, and subjected to alcohol precipitation for 24 h. The supernatant was taken and concentrated under reduced pressure to obtain an extractum. In addition, fresh Cortex Mori and Folium Mori (Sangteyou No.2) were prepared and extracted. The extraction method and parameters were as same as those described above.

In the resulting Ramulus Mori extract, the alkaloids content was 98% by mass, the polysaccharides content was 0.2% by mass, the flavonoids content was 0.05% by mass, and the amino acids content was 0 by mass. In the alkaloids, the 1-DNJ content was 99% by mass, the FAG content was 0.5% by mass, and the DAB content was 0.4% by mass.

In the resulting Cortex Mori extract, the alkaloids content was 95% by mass, the polysaccharides content was 2% by mass, the flavonoids content was 0.1% by mass, and the amino acids content was 1% by mass. In the alkaloids, the 1-DNJ content was 96% by mass, the FAG content was 1.5% by mass, and the DAB content was 1.4% by mass.

In the resulting Folium Mori extract, the alkaloids content was 90% by mass, the polysaccharides content was 4% by mass, the flavonoids content was 0.1% by mass, and the amino acids content was 3% by mass. In the alkaloids, the 1-DNJ content was 91% by mass, the FAG content was 3.1% by mass, and the DAB content was 2.8% by mass.

### Preparative Example 3 of Mulberry Extract

1000 kg of fresh Ramulus Mori (Morus atropurpurea Roxb) were weighed and crushed, then added with 11500L of water, and extracted by heating reflux for 2 h. The extracting solutions were combined, and filtered to remove insoluble matters, thereby obtaining a crude extraction solution. The crude extraction solution was centrifuged to remove impurities, then concentrated through a reverse osmosis membrane until the solid content reached 1% by mass, and served as a loading solution for the cation resin column.

300 kg of D001-type macroporous and strongly acidic styrene-based cation resin was filled in the column. The cation resin was activated according to the method described in Preparative Example 1. The concentrated crude extraction solution was loaded, and eluted with 5000L of 0.04 mol/L ammonium nitrate at an elution speed of 5 BV/h. The effluent was detected with 20% silicotungstic acid, and started to collect when a white precipitate was generated. The collection was terminated when the collection solution reached 1000L.

The collection solution resulting from the cation resin column separation was concentrated through a nanofiltration membrane, and concentrated under reduced pressure to obtain an extractum.

In the resulting Ramulus Mori extract, the alkaloids content was 15% by mass, the polysaccharides content was 20% by mass, the flavonoids content was 7% by mass, and the amino acids content was 45% by mass. In the alkaloids, the 1-DNJ content was 55% by mass, the FAG content was 23% by mass, and the DAB content was 10% by mass.

### Preparative Example 4 of Mulberry Extract

333 kg of dry Ramulus Mori (Yuesang No.11) were weighed and crushed, then added with 4000L of water, and extracted twice by heating reflux, with each reflux for 1 h. The extracting solutions were combined, filtered, and concentrated until 1 kg of crude drug/L was obtained.

150 kg of D113-type macroporous and weakly acidic phenylpropene-based cation resin was filled in the column, washed with 2 mol/L hydrochloric acid solution until the pH of the eluate was 4.5; washed with 1 mol/L sodium hydroxide solution until the pH of the eluate was 8.5; washed with 2 mol/L hydrochloric acid solution until the pH of the eluate was 4.5; and then rinsed with 5 times column volume of deionized water to complete the activation. The concentrated extracting solution was loaded, and then eluted with 1000L of 2.5 mol/L ammonia water at an elution speed of 6 BV/h. The eluate was collected when the effluent from the cation resin column was detected to be at pH > 7. When the collection solution was up to 900L, the collection was stopped. The collection solution was purified directly over the anion resin column.

125 kg of D218-type macroporous and strongly alkaline acrylic-based anion resin was filled in the column, washed with 1.5 mol/L sodium hydroxide solution until the pH of the eluate was 9.0; washed with 1.5 mol/L hydrochloric acid solution until the pH of the eluate was 3.5; washed with 1.5 mol/L sodium hydroxide solution until the pH of the eluate was 9.0 to complete the activation. The eluate collected from the cation resin was loaded onto the anion resin. The effluent at pH > 8 was collected and the collection was terminated when the effluent was up to 870L.

The collection solution resulting from the anion resin column separation was filtered via a micro-filtration membrane to remove impurities, and then concentrated through the reverse osmosis membrane. The specific gravity of the concentrated liquid was 1.1. It was transferred to an alcohol precipitation tank, and 15 kg of anhydrous ethanol was added when the stirring paddle was at 400 rpm. After adding the ethanol, the solution was stopped stirring, and subjected to alcohol precipitation for 24 h. The supernatant was taken and concentrated under reduced pressure to obtain a Ramulus Mori extractum. The sample content was as follows: the alkaloids content was 80% by mass, the polysaccharides content was 5% by mass, the flavonoids content was 0.1% by mass, and the amino acids content was 4% by mass. In the alkaloids, the 1-DNJ content was 75% by mass, the FAG content was 12% by mass, and the DAB content was 10% by mass.

### Preparative Example 5 of Mulberry Extract

400 kg of dry Ramulus Mori (Yuesang No.11) were weighed and crushed, then added with 4000L of water, and extracted twice by heating reflux, with each reflux for 1 h. The extracting solutions were combined, filtered, and concentrated until 1 kg of crude drug/L was obtained.

62.5 kg of D218-type macroporous and strongly alkaline acrylic-based anion resin was filled in the column, washed with 1.5 mol/L sodium hydroxide solution until the pH of the eluate was 9.0; washed with 1.5 mol/L hydrochloric acid solution until the pH of the eluate was 3.5; washed with 1.5 mol/L sodium hydroxide solution until the pH of the eluate was 9.0 to complete the activation. The concentrated solution collected from extraction was loaded onto the anion resin, and the effluent was collected.

The collection solution resulting from the anion resin column separation was filtered via a micro-filtration membrane to remove impurities, then concentrated through the reverse osmosis membrane, and further concentrated under reduced pressure and dried to obtain Ramulus Mori extractum. The sample content was as follows: the alkaloids content was 3% by mass, the polysaccharides content was 70% by mass, the flavonoids content was 10% by mass, and the amino acids content was 10% by mass. In the alkaloids, the 1-DNJ content was 68% by mass, the FAG content was 17% by mass, and the DAB content was 8% by mass.

### Preparative Example 6 of Mulberry Extract

1500 kg of fresh Ramulus Mori (*Morus serrata* Roxb. Yuesang No.11) were weighed and crushed, then added with 6000L of water, and extracted by heating reflux for 2 h. The extracting solutions were combined, and filtered to remove insoluble matters, thereby obtaining a crude extraction solution. The crude extraction solution was concentrated by heating until the solid content reached 4% by mass, held at 50°C, and served as a loading solution for the cation resin column.

100 kg of D113-type macroporous and weakly acidic phenylpropene-based cation resin was filled in the column, washed with 2 mol/L hydrochloric acid solution until the pH of the eluate was 4.5; washed with 1 mol/L sodium hydroxide solution until the pH of the eluate was 8.5; washed with 2 mol/L hydrochloric acid solution until the pH of the eluate was 4.5; and then rinsed with 5 times column volume of deionized water to complete the activation. The concentrated extracting solution was loaded, and then eluted with 1000L of 2.5 mol/L ammonia water at an elution speed of 6 BV/h. The eluate was collected when the effluent from the cation resin column was detected to be at pH > 7. When the collection solution was up to 900L, the collection was stopped. The collection solution was purified directly over the anion resin column.

62.5 kg of D218-type macroporous and strongly alkaline acrylic-based anion resin was filled in the column, washed with 1.5 mol/L sodium hydroxide solution until the pH of the eluate was 9.0; washed with 1.5 mol/L hydrochloric acid solution until the pH of the eluate was 3.5; washed with 1.5 mol/L sodium hydroxide solution until the pH of the eluate was 9.0 to complete the activation. The eluate collected from the cation resin was loaded onto the anion resin. The effluent was collected and the collection was terminated when the effluent was up to 870L. The effluent was concentrated under reduced pressure to obtain Ramulus Mori extractum, in which the alkaloids content was 30% by mass, the polysaccharides content was 35% by mass, the flavonoids content was 2% by mass, and the amino acids content was 25% by mass. In the alkaloids, the 1-DNJ content was 62% by mass, the FAG content was 20% by mass, and the DAB content was 13% by mass.

### Preparative Example 7 of Mulberry Extract

1000 kg of fresh Ramulus Mori (*Morus serrata* Roxb. Yuesang No.11) were weighed and crushed, then added with 4000L of water, and extracted by heating reflux for 2 h. The extracting solutions were combined, and filtered to remove insoluble matters, thereby obtaining a crude extraction solution. The crude extraction solution was concentrated by heating until the solid content reached 4% by mass, held at 50°C, and served as a loading solution for the cation resin column.

100 kg of D113-type macroporous and weakly acidic phenylpropene-based cation resin was filled in the column, washed with 2 mol/L hydrochloric acid solution until the pH of the eluate was 4.5; washed with 1 mol/L sodium hydroxide solution until the pH of the eluate was 8.5; washed with 2 mol/L hydrochloric acid solution until the pH of the eluate was 4.5; and then rinsed with 5 times column volume of deionized water to complete the activation. The concentrated extracting solution was loaded, and then eluted with 1000L of 2.5 mol/L ammonia water at an elution speed of 6 BV/h. The eluate was collected when the effluent from the cation resin column was detected to be at pH > 7. When the collection solution was up to 900L, the collection was stopped. The collection solution was purified directly over the anion resin column.

62.5 kg of D218-type macroporous and strongly alkaline acrylic-based anion resin was filled in the column, washed with 1.5 mol/L sodium hydroxide solution until the pH of the eluate was 9.0; washed with 1.5 mol/L hydrochloric acid solution until the pH of the eluate was 3.5; washed with 1.5 mol/L sodium hydroxide solution until the pH of the eluate was 9.0 to complete the activation. The eluate collected from the cation resin was loaded onto the anion resin. The effluent was collected and the collection was terminated when the effluent was up to 870L. The effluent was concentrated under reduced pressure to obtain Ramulus Mori extractum, in which the alkaloids content was 40% by mass, the polysaccharides content was 25% by mass, the flavonoids content was 0.5% by mass, and the amino acids content was 25% by mass. In the alkaloids, the 1-DNJ content was 57% by mass, the FAG content was 24% by mass, and the DAB content was 16% by mass.

### Preparative Example 8 of Mulberry Extract

333 kg of dry Ramulus Mori (Yuesang No.11) were weighed and crushed, then added with 4000L of water, and extracted twice by heating reflux, with each reflux for 1 h. The extracting solutions were combined, filtered, and concentrated until 1 kg of crude drug/L was obtained.

150 kg of D113-type macroporous and weakly acidic phenylpropene-based cation resin was filled in the column, washed with 2 mol/L hydrochloric acid solution until the pH of the eluate was 4.5; washed with 1 mol/L sodium hydroxide solution until the pH of the eluate was 8.5; washed with 2 mol/L hydrochloric acid solution until the pH of the eluate was 4.5; and then rinsed with 5 times column volume of deionized water to complete the activation. The concentrated extracting solution was loaded, and then eluted with 1000L of 2.5 mol/L ammonia water at an elution speed of 6 BV/h. The eluate was collected when the effluent from the cation resin column was detected to be at pH > 7. When the collection solution was up to 900L, the collection was stopped. The collection solution was purified directly over the anion resin column.

62.5 kg of D218-type macroporous and strongly alkaline acrylic-based anion resin was filled in the column, washed with 1.5 mol/L sodium hydroxide solution until the pH of the eluate was 9.0; washed with 1.5 mol/L hydrochloric acid solution until the pH of the eluate was 3.5; washed with 1.5 mol/L sodium hydroxide solution until the pH of the eluate was 9.0 to complete the activation. The eluate collected from the cation resin was loaded onto the anion resin. The effluent at pH > 8 was collected and the collection was terminated when the effluent was up to 870L.

The collection solution resulting from the anion resin column separation was filtered via a micro-filtration membrane to remove impurities, and then concentrated through the reverse osmosis membrane. The specific gravity of the concentrated liquid was 1.1. It was transferred to an alcohol precipitation tank, and 15 kg of anhydrous ethanol was added when the stirring paddle was at 400 rpm. After adding the ethanol, the solution was stopped stirring, and subjected to alcohol precipitation for 24 h. The supernatant was taken and concentrated under reduced pressure to obtain a Ramulus Mori extractum. The sample content was as follows: the alkaloids content was 63% by mass, the polysaccharides content was 23% by mass, the flavonoids content was 1% by mass, and the amino acids content was 5% by mass. In the alkaloids, the 1-DNJ content was 61.9% by mass, the FAG content was 16.6% by mass, and the DAB content was 11.1% by mass.

### Preparative Example 9 of Mulberry Extract

1000 kg of fresh Ramulus Mori (Yuesang No.11) were weighed and crushed, then added with 4000L of water, and extracted by heating reflux for 2 h. The extracting solutions were combined, and filtered to remove insoluble matters, thereby obtaining a crude extraction solution. The crude extraction solution was concentrated by heating until the solid content reached 4% by mass, held at 50°C, and served as a loading solution for the cation resin column.

120 kg of D113-type macroporous and weakly acidic phenylpropene-based cation resin was filled in the column, washed with 2 mol/L hydrochloric acid solution until the pH of the eluate was 4.5; washed with 1 mol/L sodium hydroxide solution until the pH of the eluate was 8.5; washed with 2 mol/L hydrochloric acid solution until the pH of the eluate was 4.5; and then rinsed with 5 times column volume of deionized water to complete the activation. The concentrated extracting solution was loaded, and then eluted with 1000L of 2.5 mol/L ammonia water at an elution speed of 6 BV/h. The eluate was collected when the effluent from the cation resin column was detected to be at pH > 7. When the collection solution was up to 900L, the collection was stopped. The collection solution was purified directly over the anion resin column.

45 kg of D218-type macroporous and strongly alkaline acrylic-based anion resin was filled in the column, washed with 1.5 mol/L sodium hydroxide solution until the pH of the eluate was 9.0; washed with 1.5mol/L hydrochloric acid solution until the pH of the eluate was 3.5; and washed with 1.5 mol/L sodium hydroxide solution until the pH of the eluate was 9.0 to complete the activation. The eluate collected from the cation resin was loaded onto the anion resin. The effluent was collected and the collection was terminated when the effluent was up to 870L.

The collection solution resulting from the anion resin column separation was filtered via a micro-filtration membrane to remove impurities, and then concentrated through the reverse osmosis membrane. The specific gravity of the concentrated liquid was 1.1. It was transferred to an alcohol precipitation tank, and 15 g of anhydrous ethanol was added when the stirring paddle was at 300 rpm. After adding the ethanol, the solution was stopped stirring, and subjected to alcohol precipitation for 24 h. The supernatant was taken and concentrated under reduced pressure to obtain an extractum. The sample content was as follows: the alkaloids content was 70% by mass, the polysaccharides content was 20% by mass, the flavonoids content was 0.6% by mass, and the amino acids content was 5% by mass. In the alkaloids, the 1-DNJ content was 70% by mass, the FAG content was 13% by mass, and the DAB content was 10% by mass.

### II. Pharmacodynamic Experiments of Mulberry Extract

### Test Example 1: Effect of Mulberry Extract on Spermatogenesis in Normal Rats

### 1. Test sample

Mulberry extract obtained in Preparative Example 1.

### 2. Preparation of solution for administration

A given amount of the test sample was weighed into a suitable beaker, and an appropriate volume of vehicle was added. The mixture was subjected to ultrasonication (300 W, 40 KHZ) for approximately 10 min until the test sample was completely dissolved. The reaction solution was allowed to stand to room temperature, and then transferred to a graduated cylinder and adjusted to the required volume to obtain the formulation solution at the required concentration.

### 3. Animal administration and sample collection

Forty 5- to 7-week-old male SD rats (138 to 183 g) were randomly divided into a vehicle control group and three mulberry extract dosing groups (administered at doses of 100 mg/kg/d, 200 mg/kg/d, and 500 mg/kg/d, respectively based on the total alkaloids). The rats were administered by gavage at a dose of 10 mL/kg once daily. At week 4 after administration, the male rats were euthanized, and their testes and epididymides were collected.

### 4. Detection methods

### Sperm Count:

One epididymis was taken and placed into 1 mL of 0.9% sodium chloride injection, and the epididymal tissue was minced and incubated at 37°C to prepare a sperm suspension. The sperm suspension was then loaded into a hemocytometer, and the number of sperm was counted under a microscope. Subsequently, the total sperm count was calculated based on the dilution factor.

### Sperm Motility Examination:

Slide preparation: The cauda epididymis was cut open with ophthalmic scissors. The contents of the cauda epididymis were mixed evenly with 0.9% sodium chloride injection to prepare a sperm suspension. Two drops of the sperm suspension were dropped onto a glass slide. The above operations were carried out at a constant temperature of 37°C.

Observation: The swimming status of approximately 200 sperm was randomly recorded under a high-power microscope. The swimming status of sperm was assessed according to the following criteria: I. good motility: sperm swam rapidly in a straight line; II. moderate motility: sperm were relatively active; III. poor motility: sperm moved sluggishly or spun in place; IV. dead sperm: sperm exhibited normal morphology but were immotile.

### 5. Test results

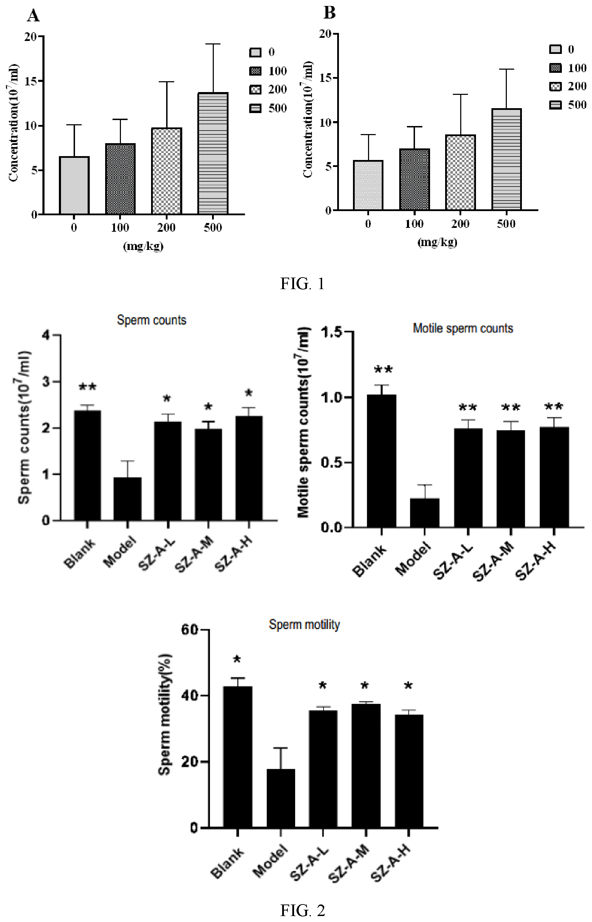

Compared with the vehicle control group, each mulberry extract dose group showed an increase in both sperm count and motile sperm count in male rats (Table 1 and FIG. 1).

**Table 1 Sperm Function**

| Group | Dose (mg/k g) | Number of animals (n) | Total sperm count (×10⁷/mL) | Motile sperm count (×10⁷/mL) | Motility distribution (%) | | | | Motility rate (%) |
|---|---|---|---|---|---|---|---|---|---|
| | | | | | Grade I | Grade II | Grade III | Grade IV | |
| Vehicle control group | 0 | 10 | 6.6±3.5 | 5.7±2.9 | 45.2 | 20.6 | 21.7 | 12.5 | 87.5 |
| | 100 | 10 | 8.0±2.7 | 7.0±2.5 | 46.0 | 18.3 | 22.6 | 13.0 | 87.0 |
| Mulberry extract | 200 | 10 | 9.8±5.1 | 8.6±4.5 | 44.7 | 19.3 | 23.4 | 12.5 | 87.5 |
| | 500 | 10 | 13.7±5.5 | 11.5±4.5 | 34.4 | 23.8 | 26.0 | 15.8 | 84.2 |

| | | | | | | | | | |
|---|---|---|---|---|---|---|---|---|---|
| Note: Dilution method: One epididymis was taken, minced, and then added with 1 mL of normal saline. 50 µL of the solution was measured and diluted to 1 mL, and then 100 µL of the solution was measured and diluted to 1 mL. Thereafter, the sperm was counted under a microscope. The number of sperm (M) in the four large squares around the hemocytometer was counted. Calculation formula: M/0.4 × dilution factor × 1000 = sperm count/mL. | | | | | | | | | |

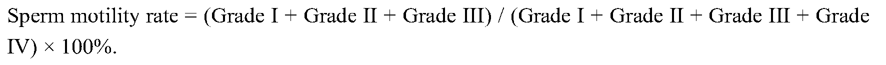

Sperm motility rate = (Grade I + Grade II + Grade III) / (Grade I + Grade II + Grade III + Grade IV) × 100%.

Table 1 and FIG. 1 showed that after healthy male SD rats were administered with mulberry extract by gavage for four consecutive weeks, the total sperm count and the motile sperm count increased, indicating that mulberry extract could promote spermatogenesis in male rats.

### Test Example 2: GAN-Induced Reproductive Dysfunction Model in Male Mice

### 1. Test sample

Ramulus Mori extract (SZ-A) obtained in Preparative Example 8.

### 2. The groups in the experiment were as follows.

**Table 2**

| Group | Abbrev. | Number of animals (n) | Dose (based on total alkaloids, mg/kg/d) | Mode of administration | Diets |
|---|---|---|---|---|---|
| Blank group | Blank | 10 | 0 | | Chow (normal diet) |
| Model group | Model | 10 | 0 | | GAN |
| SZ-A | SZ-A-L | 10 | 200 | Gavage | |
| | SZ-A-M | 10 | 300 | | |
| | SZ-A-H | 10 | 400 | | |

| | | | | | |
|---|---|---|---|---|---|
| Note: Gubra-Amylin NASH (GAN) was a high-fat, high-cholesterol, high-fructose diet containing 40% saturated fat, 22% fructose, and 2% cholesterol. | | | | | |

### 3. Experimental Method

Fifty healthy male C57BL/6J mice with glossy fur were selected and fed with maintenance diet for one week of acclimatization and observation. After confirmation that the animals were well acclimated, modeling was initiated. The mice in the model group were fed a GAN diet (Research Diets D09100310), while the mice in the control group continued to receive the maintenance diet for 20 weeks to establish male mouse models of reproductive dysfunction. The mice were divided into 5 groups: a control group, a model group, and low-, medium-, and high-dose Ramulus Mori extract groups (designated as SZ-A-L, SZ-A-M, and SZ-A-H, respectively), with 10 mice in each group. In each test group, the mice were administered according to the dose and mode of administration specified in the table. The mice in the control group and model group were administered with an equal volume of normal saline. At day 21 after administration, samples were collected for pharmacodynamic evaluation.

### 4. Detection methods

### 1) Sperm count detection

After the mouse was dissected, one epididymis was taken and placed in normal saline (1 mL), and the epididymal tissue was minced to release sperm. The sample was then incubated on a shaker at 37.6°C for 5 min. The sperm count was immediately determined using a hemocytometer.

### 2) Motile sperm count detection

The cauda epididymis was cut open with ophthalmic scissors. The contents of the cauda epididymis were mixed with 0.9% sodium chloride injection to prepare a sperm suspension. Two drops of the sperm suspension were dropped onto a glass slide. The above operations were carried out at a constant temperature of 37°C. Observation was carried out under a high-power microscope, and the swimming status of 200 sperm was randomly recorded. The motile sperm count and sperm motility were calculated. Sperm motility = motile sperm count / sperm count.

3) The testosterone (T) level was detected by Elisa.

4) The levels of androgen synthases (STAR, CYP17A1) and androgen receptor (AR) were detected by Western.

5) Histopathology: HE staining was performed to observe the structure of seminiferous tubules in testicular tissue as well as the morphology and number of spermatogenic cells, and to observe sperm maturation and spermatocyte classification.

### 5. Results analysis

### (1) Total sperm count, motile sperm count, and sperm motility

The results in FIG. 2 showed that compared with the control group, the total sperm count, motile sperm count, and sperm motility in the model group were all significantly reduced. After treatment with SZ-A, the low-, medium-, and high-dose SZ-A groups all showed significantly increased total sperm count, motile sperm count, and sperm motility, and particularly the improved effect on the total sperm count, which was nearly restored to the normal level.

### (2) Testosterone level

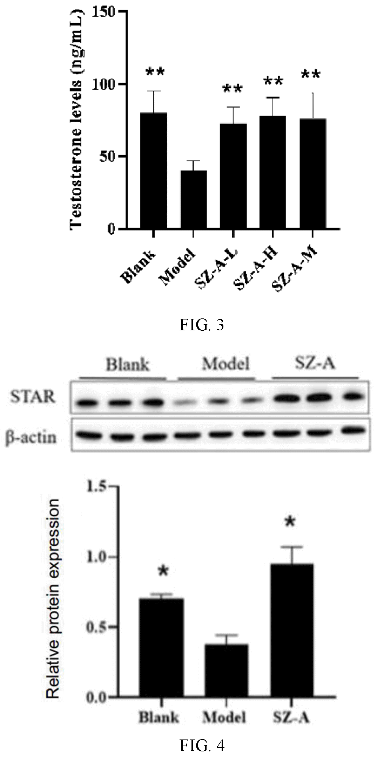

The results in FIG. 3 showed that compared with the control group, the testosterone levels in the testicular tissue of the model group were significantly decreased. After treatment with SZ-A, the low-, medium-, and high-dose SZ-A groups all showed significantly increased testosterone levels in the testicular tissue.

### (3) STAR, CYP17A1, and AR levels

The results shown in FIGS. 4-6 showed that in the high-dose SZ-A group, the expression of STAR as a rate-limiting enzyme for testosterone synthesis, CYP17A1 as a key enzyme for testosterone synthesis, and an androgen receptor AR was significantly increased.

### (4) Seminiferous tubule structure in testicular tissue

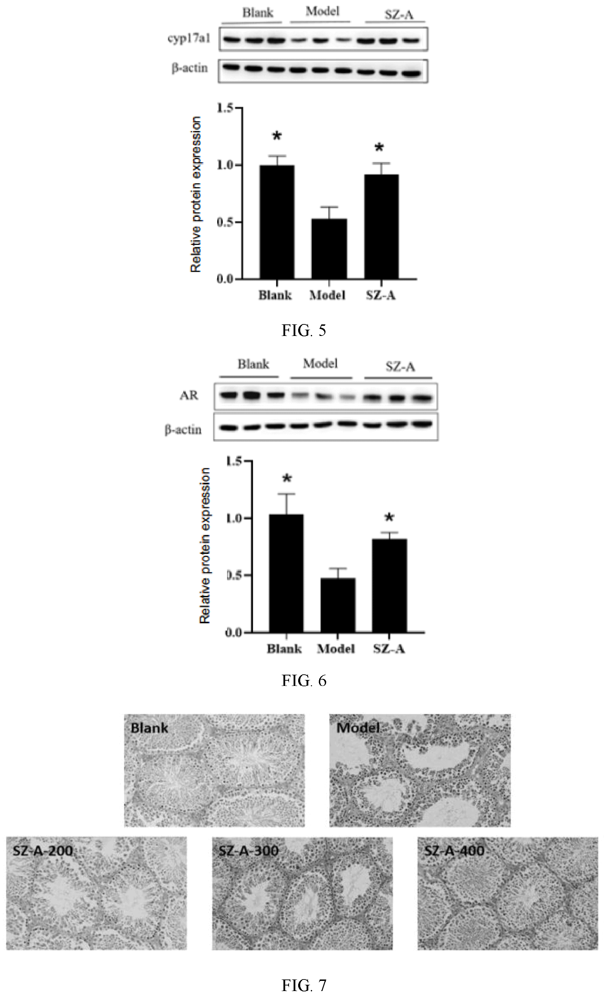

The results in FIG. 7 showed that in the Blank group, the seminiferous tubules of mouse testes were tightly connected, the spermatogenic cells at all stages were orderly arranged, and no obvious abnormalities in cell morphology were observed. In the Model group, the seminiferous tubules of mouse testes were loosely connected, and vacuolar degeneration of Leydig cells and inflammatory cell infiltration were observed; the spermatogenic cells at all stages in the seminiferous tubules were loosely connected and disorganized, the number of layers of spermatogenic cells was reduced, and some spermatogenic cells were detached. Compared with the Model group, all SZ-A dose groups showed a significantly improved structure of the seminiferous tubules and status of interstitial cells, alleviated inflammatory cell infiltration, and increased number and quantity of spermatogenic cells at all stages, with tighter connections and neater arrangement.

### (5) Number of spermatogenic cells

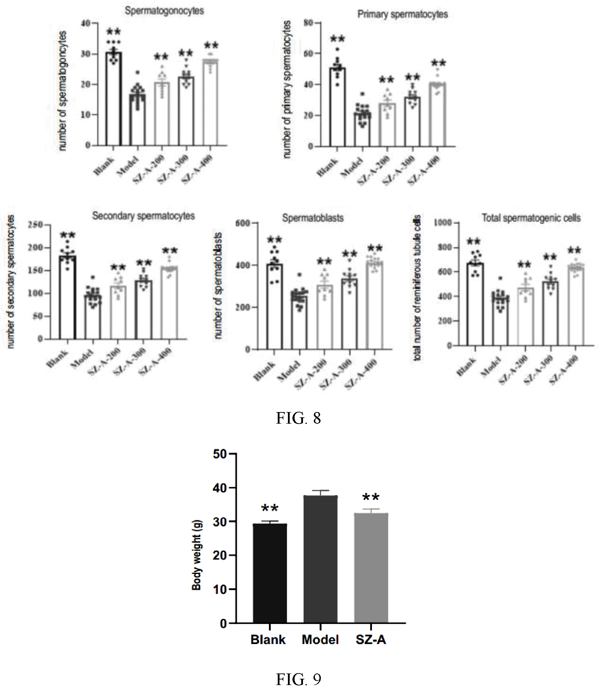

The results in FIG. 8 showed that compared with the Blank group, the spermatogonocytes, primary spermatocytes, secondary spermatocytes, spermatoblasts, and total spermatogenic cells within a single seminiferous tubule in the Model group were significantly reduced. After treatment with SZ-A, the number of spermatogenic cells at all stages was significantly increased in a dose-dependent manner in all dose groups. Among them, the improved effect on the number of spermatogenic cells in the high-dose SZ-A group was close to that in the normal group.

### Test Example 3: HFD-induced C57BL/6J Mouse Model of Obesity-Related Reproductive Dysfunction

Forty-five healthy 6-week-old male C57BL/6J mice were randomly divided into a normal group (Chow), a high-fat diet model group (HFD), and mulberry extract (SZ-A) groups, with 9 mice in each group. The mulberry extract was the one prepared in Preparative Example 9. The mice in the normal group were fed a basal diet, while the mice in the model group and SZ-A groups were fed with a high-fat diet (Research Diet D12492, 60 kcal% fat). After feeding for 8 weeks, the mice in each test group were treated with a corresponding dose of the test drug for 6 consecutive weeks. During the treatment period, the mice continued to be fed with the high-fat diet, and animal body weight as well as food and water intake were monitored. The model group received normal saline. After the completion of the experiment, samples were collected for pharmacodynamic evaluation.

**Table 3 Experimental Grouping and Administration Method**

| Type | Group | Dose (based on alkaloids) |
|---|---|---|
| Normal mice | Blank | 0 |
| Model | Model | 0 |
| SZ-A | SZ-A | 400 mg/kg/d |

### Detection indicators:

(1) The total sperm count, motile sperm count, and sperm motility of mice were detected by the same methods as those described in Test Example 2.
(2) Histopathology of penile tissue in mouse model: HE staining was performed to observe the corpus cavernosum structure of penile tissue and to evaluate the improvements in the average thickness of the corpus cavernosum scaffolds, connection density, degree of separation, and mean intercept length of the sinusoidal lacunae.

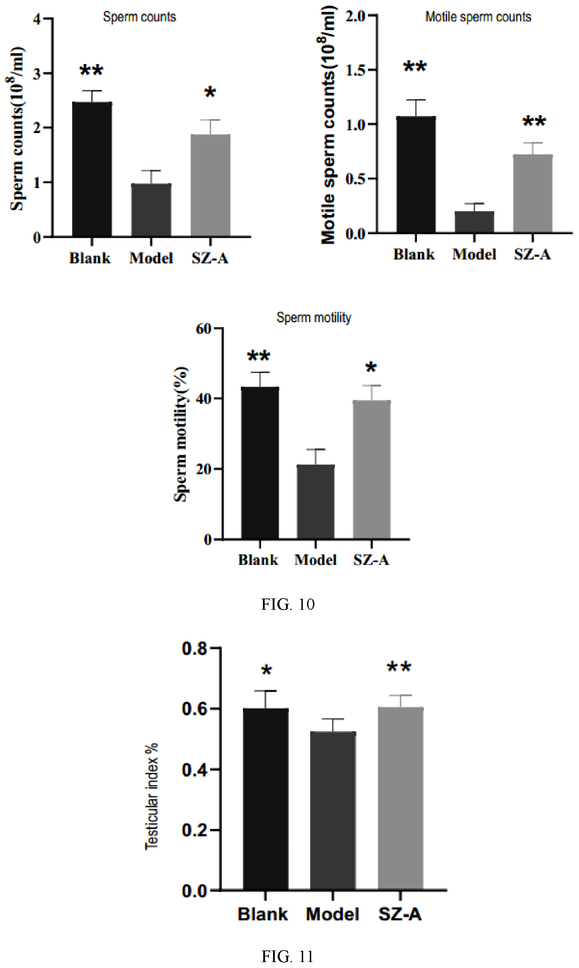

The results in FIGS. 9-12 showed that the model group exhibited the decreased total sperm count, motile sperm count, and sperm motility of mice, the significantly increased body weight, the reduced testicular index, and the deteriorated corpus cavernosum structure, characterized by abundant free cavernous scaffolds in the central region, significantly reduced overall number of branches of the corpus cavernosum scaffolds, and locally fused sinusoidal lacunae with a markedly enlarged area. Compared with the model group, SZ-A could significantly increase the total sperm count, motile sperm count, and sperm motility of mice, reduce the body weight, and increase the testicular index to a level almost restored to that in the normal group. Moreover, the cavernous scaffolds in the penile corpus cavernosum was in a reticular distribution, the overall number of branches of the corpus cavernosum scaffolds was slightly reduced, and the scaffolds were thick and compact with relatively small inter-scaffold spaces.

### Test Example 4. Diabetes-Related Sexual Dysfunction Model in Apoe Mice induced by STZ + High-Fat High-Cholesterol Diet

After being acclimatized for one week, thirty eight-week-old Apoe mice were fed with a high-fat high-cholesterol (HFHC) diet for 5 weeks. At week 6, the mice received a single intraperitoneal injection of STZ (streptozotocin) (100 mg/kg/d) to construct a mouse model of glycolipid metabolism disorders. Meanwhile, ten eight-week-old C57 mice were selected and acclimatized for one week, then fed with a normal diet for 5 weeks, and received a single intraperitoneal injection of an equal volume of vehicle at week 6, serving as the normal control group.

After successful modeling, the model mice were randomly divided into 3 groups: a model group, and low-dose (150 mg/kg/day) and high-dose (300 mg/kg/day) groups of SZ-A obtained in Preparative Example 8. The mice were administered for 6 weeks according to the table below, and then samples were collected for pharmacodynamic evaluation.

**Table 4 Animal Grouping and Mode of Administration**

| Group | Abbrev. | Number of animals (n) | Dose (based on total alkaloids, mg/kg/d) | Mode of administration | Diets |
|---|---|---|---|---|---|
| Blank group | Blank | 10 | 0 | | Chow |
| Model group | Model | 10 | 0 | Gavage | HFHC |
| SZ-A | SZ-A-L | 10 | 150 | | |
| | SZ-A-H | 10 | 300 | | |

### Detection indicators:

(1) The total sperm count, motile sperm count, and sperm motility of mice were detected by the same methods as those described in Test Example 2.

### Histopathology:

(2) Effect of SZ-A on testicular pathology: HE staining was performed to observe the structure of seminiferous tubules in testicular tissue as well as the morphology and number of spermatogenic cells, and to observe sperm maturation and spermatocyte classification.
(3) HE staining was performed to observe the corpus cavernosum structure of penile tissue, and to evaluate the improvements in the average thickness of the corpus cavernosum scaffolds, connection density, degree of separation, and mean intercept length of the sinusoidal lacunae.

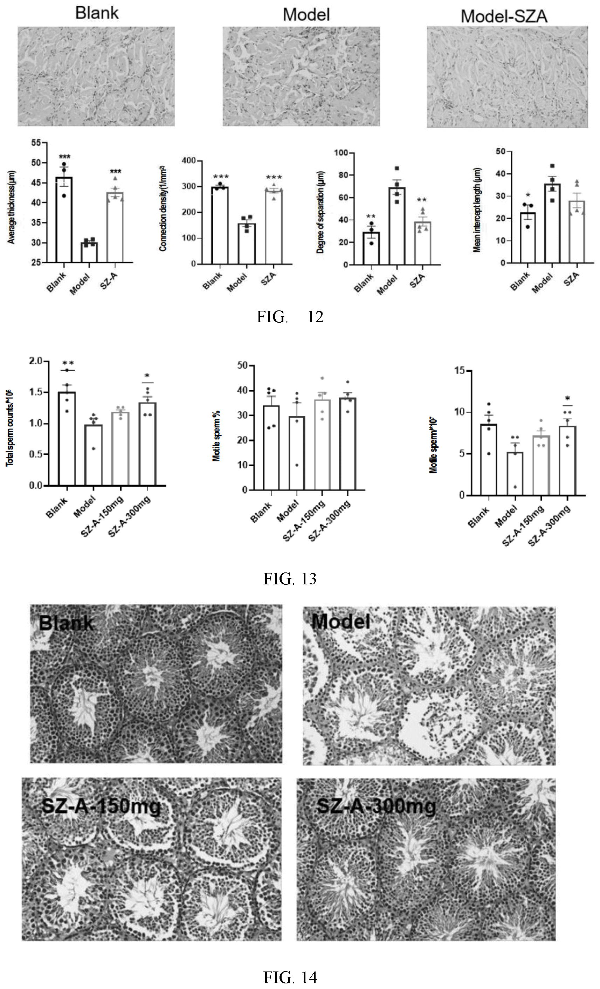

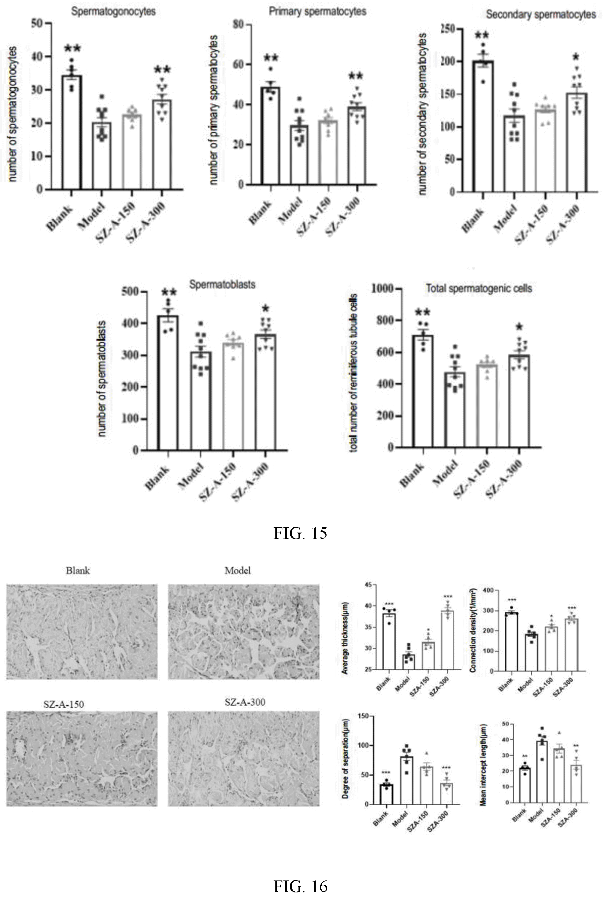

Results analysis:
(1) The results in FIG. 13 showed that in the model group, the total sperm count, motile sperm count, and sperm motility of mice were decreased. Compared with the model group, SZ-A could significantly increase the total sperm count, motile sperm count, and sperm motility of mice.
(2) The results in FIG. 14 showed that in the Blank group, the seminiferous tubules of mouse testes were tightly connected, the spermatogenic cells at all stages were orderly arranged, and no obvious abnormalities in cell morphology were observed. In the Model group, the seminiferous tubules of mouse testes were loosely connected, and vacuolar degeneration of Leydig cells and inflammatory cell infiltration were observed; the spermatogenic cells at all stages in the seminiferous tubules were loosely connected and disorganized, the number of layers of spermatogenic cells was reduced, and some spermatogenic cells were detached. Compared with the Model group, all SZ-A dose groups showed a significantly improved structure of the seminiferous tubules of testes and status of the interstitial cells, reduced inflammatory cell infiltration, and increased number and quantity of spermatogenic cells at all stages, with tighter connections and neater arrangement.
   The results in FIG. 15 showed that compared with the Blank group, the number of spermatogonocytes, primary spermatocytes, secondary spermatocytes, spermatoblasts, and total spermatogenic cells within a single seminiferous tubule in the model group were significantly reduced. After treatment with SZ-A, the number of spermatogenic cells at all stages was significantly increased in a dose-dependent manner in all dose groups. Among them, the improved effect on the number of spermatogenic cells in the high-dose SZ-A group was close to that in the normal group.
(3) HE staining was performed to observe the structure of corpus cavernosum of penile tissue and the histopathological results were as shown in FIG. 16. In the model group, the structure of the penile corpus cavernosum was deteriorated, which was evidenced by abundant free cavernous scaffolds in the central region, significantly reduced overall number of branches of the corpus cavernosum scaffolds, and locally fused sinusoidal lacunae with a markedly enlarged area. Compared with the model group, the SZ-A groups showed that the cavernous scaffolds in the penile corpus cavernosum was in a reticular distribution, the overall number of branches of the corpus cavernosum scaffolds was slightly reduced, and the scaffolds were thick and compact with relatively small inter-scaffold spaces, and that the average thickness of cavernous scaffolds, connection density, degree of separation, and mean intercept length of the sinusoidal lacunae were all improved.

### Test Example 5: Effect of SZ-A on PA (Palmitic Acid)-Induced Testicular Leydig Cell Injury

TM3 cells (normal mouse testicular Leydig cells) were seeded in a 6-well plate at 5*10⁵/mL and cultured overnight. Subsequently, the cells were divided into a blank group, a model group, and SZ-A dose groups (as grouped below). The model group and SZ-A dosing groups were treated with 400 µmol/L of PA, and the SZ-A groups were additionally treated with the drug. After 24-hour incubation, pharmacodynamic evaluation was performed.
Group 1: control group;
Group 2: model group (PA group);
Group 3: dosing group, the drug was 200 µg/mL of Ramulus Mori extract in Preparative Example 1;
Group 4: dosing group, the drug was 200 µg/mL of Ramulus Mori extract in Preparative Example 2;
Group 5: dosing group, the drug was 200 µg/mL of Ramulus Mori extract in Preparative Example 3;
Group 6: dosing group, the drug was 200 µg/mL of Ramulus Mori extract in Preparative Example 4;
Group 7: dosing group, the drug was 200 µg/mL of Ramulus Mori extract in Preparative Example 5;
Group 8: dosing group, the drug was 200 µg/mL of Ramulus Mori extract in Preparative Example 7.
   1) STAR as a rate-limiting enzyme for testosterone synthesis, and CYP17A1 and 17β-HSD as key enzymes for testosterone synthesis were detected by Western following SZ-A treatment.

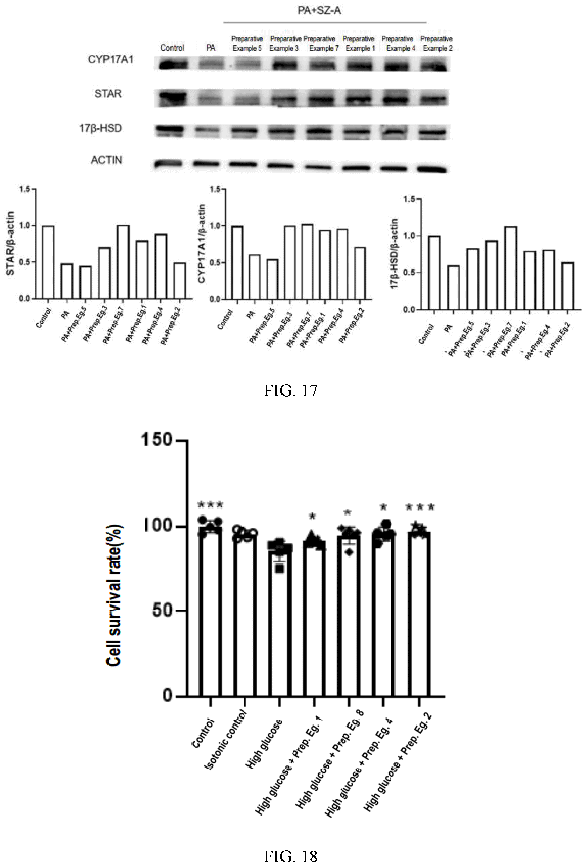

The results in FIG. 17 showed that after the testicular Leydig cells were induced by PA, the expression of multiple androgen synthesis-related enzymes CYP17A1, STAR, and 17β-HSD were reduced in the model group. However, after intervention with different amounts of SZ-A, the expression of androgen synthesis-related enzymes could be enhanced.

### Test Example 6. Effect 1 of SZ-A on High Glucose-Induced Corpus Cavernosum Endothelial Cell Injury

RCCEC cells (endothelial cells of corpus cavernosum) were seeded in a 96-well plate at 5,000 cells/well and cultured overnight in a serum-free medium. The cells were divided into a control group, an isotonic control group, a model group, and SZ-A dose groups (as grouped below). The control group was cultured in endothelial cell-specific medium containing 5.55 mM glucose. The isotonic control group was cultured in endothelial cell-specific medium containing 5.55 mM glucose supplemented with 24.45 mM mannitol. The model group and SZ-A dosing groups were cultured in endothelial cell-specific medium containing 5.55 mM glucose supplemented with 24.45 mM glucose (i.e., 30 mM glucose in total). The SZ-A groups were treated with a drug. After 72-hour incubation, the cell viability was detected using the CCK-8 reagent, and the cell survival rate was calculated.
Group 1: control group: 5.55 mM glucose
Group 2: isotonic control group: 5.55 mM glucose + 24.45 mM mannitol
Group 3: model group: 30 mM glucose
Group 4: 30 mM glucose + 150 µg/ml SZ-A (in terms of extract); SZ-A was the extract in Preparative Example 1;
Group 5: 30 mM glucose + 150 µg/ml SZ-A (in terms of extract); SZ-A was the extract in Preparative Example 8;
Group 6: 30 mM glucose + 150 µg/ml SZ-A (in terms of extract); SZ-A was the extract in Preparative Example 4;
Group 7: 30 mM glucose + 150 µg/ml SZ-A (in terms of extract); SZ-A was the extract in Preparative Example 2;
The results in FIG. 18 showed that SZ-A could inhibit high glucose-induced corpus cavernosum endothelial cell injury and improve the cell survival rate.

### Test Example 7. Effect 2 of SZ-A on High Glucose-Induced Corpus Cavernosum Endothelial Cell Injury

RCCEC cells (endothelial cells of corpus cavernosum) were seeded in a 6-well plate at 3*10⁵/mL and cultured overnight in a serum-free medium. The cells were then divided into a control group, an isotonic control group, a model group, and SZ-A dose groups (as grouped below). The control group was cultured in endothelial cell-specific medium containing 5.55 mM glucose. The isotonic control group was cultured in endothelial cell-specific medium containing 5.55 mM glucose and 24.45 mM mannitol. The model group and SZ-A dosing groups were cultured in endothelial cell-specific medium containing 5.55 mM glucose supplemented with 24.45 mM glucose (i.e., 30 mM glucose in total). The SZ-A groups were treated with a drug. After 72-hour incubation, the cells were collected, and the nitric oxide content in the cells was determined.
Group 1: control group: 5.55 mM glucose
Group 2: isotonic control group: 5.55 mM glucose + 24.45 mM mannitol
Group 3: model group: 30 mM glucose
Group 4: 30 mM glucose + 150 µg/ml SZ-A (in terms of extract); SZ-A was the extract in Preparative Example 3;
Group 5: 30 mM glucose + 150 µg/ml SZ-A (in terms of extract); SZ-A was the extract in Preparative Example 1;
Group 6: 30 mM glucose + 150 µg/ml SZ-A (in terms of extract); SZ-A was the extract in Preparative Example 4;
Group 7: 30 mM glucose + 150 µg/ml SZ-A (in terms of extract); SZ-A was the Ramulus Mori extract in Preparative Example 2;

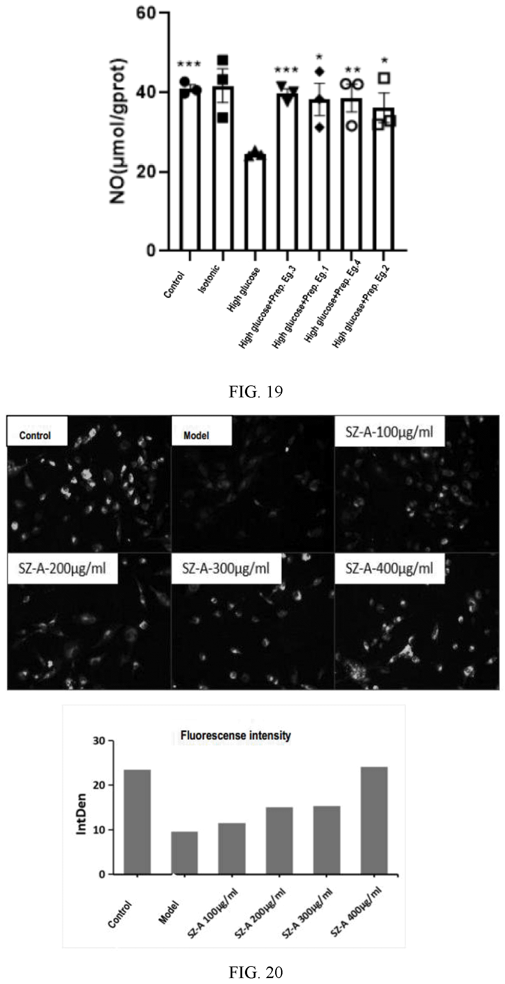

The results in FIG. 19 showed that SZ-A could inhibit high glucose-induced corpus cavernosum endothelial cell injury and improve the NO content in the cells.

### Test Example 8. Effect of SZ-A on High Insulin-Induced Endothelial Cell Injury Model

Cell Culture: human umbilical vein endothelial cells (HUVECs) were cultured in DMEM medium (10% FBS + 1% penicillin-streptomycin).

Cell Passage: Cells were digested with 0.25% trypsin, seeded into a 12-well plate at a density of 10⁵ cells/mL, and cultured in a cell culture incubator at 37°C with 5% CO₂. Subsequently, the culture medium was changed every 2 to 3 days. The cells could be sub-cultured again once they reached approximately 80% confluence.

Model construction: The passaged HUVEC cells were cultured overnight and then randomly divided into six groups: a normal group, a model group, and SZ-A groups (Ramulus Mori extract of Preparative Example 8, administered at doses of 100 µg/mL, 200 µg/mL, 300 µg/mL, and 400 µg/mL respectively based on the total alkaloids). Among them, the cells in the normal group were cultured in DMEM medium (10% FBS + 1% penicillin-streptomycin), the cells in the model group were cultured in DMEM medium (10% FBS+ 1% penicillin-streptomycin + 40 U insulin), and the cells in the SZ-A groups were cultured in DMEM medium (10% FBS + 1% penicillin-streptomycin + 40U insulin+ corresponding dose of SZ-A). The cells were cultured for 24 h, and then the mitochondrial membrane potential was detected using the Mito-Tracker method.

Mito-Tracker Deep Red FM (mitochondrial far-red fluorescent probe): When the cells were cultured to a certain density in cell culture plates or dishes, the cell culture medium was removed, and the prepared Mito-Tracker Deep Red FM working solution was added. After incubation at 37°C for 15 to 30 min, the Mito-Tracker Deep Red FM working solution was removed, and a fresh cell culture medium pre-warmed to 37°C was added. The cells were observed using a fluorescence microscope, under which mitochondria exhibited bright and intense fluorescent staining.

Results: SZ-A could increase the number of mitochondria in endothelial cells and ameliorate the mitochondrial damage, suggesting that SZ-A could significantly improve the endothelial cell function (FIG. 20).

### Test Example 9: Clinical Assessment

### 1. Experimental Protocol

### 1.1 Experimental grouping

This trial adopted a prospective, single-arm, open-label, multicenter trial design. Inclusion criteria: (1) males aged 45-70 years, married or unmarried but with a fixed sexual partner; (2) subjects diagnosed with type 2 diabetes according to the WHO criteria, with glycated hemoglobin (HbA1c) ≥ 7%; (3) subjects with clear consciousness and without cognitive abnormalities; and (4) subjects who signed the informed consent forms. Exclusion criteria: (1) history of prostate, penile, or urethral surgery or trauma; (2) congenital abnormalities of the reproductive system; (3) use of drugs that possibly caused ED within the past month; (4) major physical diseases such as severe organ dysfunction or malignant tumors; (5) history of drug abuse; and (6) psychiatric disorders. All subjects signed the informed consent forms. A total of 17 subjects were enrolled.

### 1.2 Administration method

The experimental group received oral tablets with a starting dose of one tablet per time, three times daily. After 4 weeks, the dose was escalated to two tablets per time, three times daily. The consecutive treatment duration was 48 weeks in total.

Preparation method for the tablets: The Ramulus Mori extract of Preparation Example 8 was taken, and an appropriate amount of excipients was added and blended uniformly. Water was added to prepare a soft mass, which was then granulated and dried. Magnesium stearate was added and mixed uniformly, and the mixture was compressed into tablets to obtain the Ramulus Mori extract formulation. Each tablet contained 50 mg of total alkaloids.

### 1.3 Experimental data detection and processing

1. At week 0 (before administration) and week 48 (after administration), the International Index of Erectile Function (IIEF) was used to assess the degrees of ED of the subjects. The IIEF consisted of 15 questions, each scored on a 6-point scale (0 to 5 points), with a total score of 75 points. The scoring table was as follows:

**Table 5**

| **Sexual Function over the Past 4 Weeks** | **0** | **1** | **2** | **3** | **4** | **5** |
|---|---|---|---|---|---|---|
| 1) How often were you able to get an erection during sexual activity over the past 4 weeks? | No sexual activity | Almost never or never | Less than 50% | About 50% | More than 50% | Almost 100% or 100% |
| For example: if there were 4 sexual activities and an erection was achieved each time, the result was 100%, if two of the erections were achieved, the result was 50%, and so forth. | | | | | | |
| 2) When you had erections with sexual stimulation, how often were your erections hard enough for penetration? | No sexual activity | Almost never or never | Less than 50% | About 50% | More than 50% | Almost 100% or 100% |
| 3) When you attempted intercourse, how often were you able to penetrate (enter) your partner? | No sexual activity | Almost never or never | Less than 50% | About 50% | More than 50% | Almost 100% or 100% |
| 4) How often were you able to maintain your erection after you had penetrated (entered) your partner? | No sexual activity | Almost never or never | Less than 50% | About 50% | More than 50% | Almost 100% or 100% |
| 5) How difficult was it to maintain your erection to completion of intercourse? | Did not attempt intercourse | Extremely difficult | Very difficult | Difficult | Slightly difficult | Not difficult |
| 6) How many times have you attempted sexual intercourse? | Did not attempt intercourse | One to two attempts | Three to four attempts | Five to six attempts | Seven to ten attempts | Eleven or more attempts |
| 7) When you attempted sexual intercourse, how often was it satisfactory for you? | No sexual activity | Almost never or never | Less than 50% | About 50% | More than 50% | Almost 100% or 100% |
| 8) How much have you enjoyed sexual intercourse? | No intercourse | No enjoyment at all | Not very enjoyable | Fairly enjoyable | Highly enjoyable | Very highly enjoyable |
| 9) When you had sexual stimulation or intercourse, how often did you ejaculate? | No sexual activity | Almost never or never | Less than 50% | About 50% | More than 50% | Almost 100% or 100% |
| 10) When you had sexual | | Almost never | Less than | About | More than | Almost 100% or |
| stimulation or intercourse, how often did you have the feeling of orgasm or climax? | | or never | 50% | 50% | 50% | 100% |
| 11) How often have you felt libido? | | Almost never or never | A few times | Sometimes | Most times | Almost always or always |
| 12) How would you rate your level of libido? | | Very low or none at all | low | Moderate | High | Very high |
| 13) How satisfied have you been with your overall sex life? | | Very dissatisfied | Moderately dissatisfied | Equally satisfied & dissatisfied | Moderately satisfied | Very satisfied |
| 14) How satisfied have you been with your sexual relationship with your partner? | | Very dissatisfied | Moderately dissatisfied | Equally satisfied & dissatisfied | Moderately satisfied | Very satisfied |
| 15) How do you rate your confidence that you could get and keep an erection? | | Very low | Low | Moderate | High | Very high |

| | | | | | | |
|---|---|---|---|---|---|---|
| Note: The International Index of Erectile Function (IIEF) questionnaire was initially developed during the clinical development of sildenafil to provide a more reliable assessment of erectile function status and therapeutic effects in patients with ED. Currently, IIEF-15 had become the gold standard for efficacy evaluation in ED clinical trials and had been progressively applied to studies on different etiologies of ED, various comorbidities associated with ED, diverse cultural backgrounds, or different treatment interventions. It consisted of 15 items, which were divided into five domains: erectile function (questions 1-5), satisfaction with penetration (questions 6-8), orgasm (questions 9-10), libido (questions 11-12), and overall satisfaction (questions 13-15). Patients answered each item based on their sexual activities over the past 4 weeks. The lower the IIEF-15 score, the more severe the ED (the poorer the erectile function). | | | | | | |

### 2. Statistical analysis

The SPSS 20.0 software was used for data processing. The differences before and after treatment were analyzed using the t-test.

### 3. Experimental results

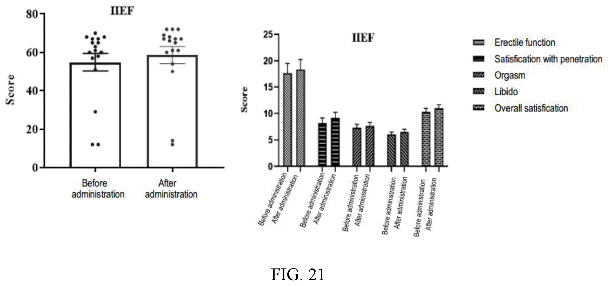

The results showed that after treatment with SZ-A, the IIEF-15 scores increased, suggesting that SZ-A could improve the erectile function. It was found that SZ-A improved all five domains of erectile function, satisfaction with penetration, orgasm, libido, and overall satisfaction (FIG. 21). Further analysis revealed that eleven of the seventeen subjects showed an increase in score after treatment with SZ-A, suggesting that the treatment rate of SZ-A for ED was approximately 65%.

## Claims

1. Use of a mulberry extract in preparation of a drug for treatment and/or prevention of male infertility, wherein, based on a weight percentage of a sum of components of the mulberry extract as 100%, the mulberry extract comprises alkaloids in an amount of 3% by weight or more (optionally comprises alkaloids in an amount of 3 to 99% by weight, further optionally comprises alkaloids in an amount of 15 to 99% by weight, further optionally comprises alkaloids in an amount of 30 to 99% by weight, further optionally comprises alkaloids in an amount of 40 to 99% by weight, further optionally comprises alkaloids in an amount of 50 to 99% by weight, or further optionally comprises alkaloids in an amount of 60 to 99% by weight), and/or
comprises polysaccharides in an amount of not more than 70% by weight (optionally comprises polysaccharides in an amount of 0.2 to 70% by weight, optionally further comprises polysaccharides in an amount of 0.2 to 50% by weight, optionally further comprises polysaccharides in an amount of 0.2 to 35% by weight, optionally further comprises polysaccharides in an amount of 0.2 to 25% by weight, optionally further comprises polysaccharides in an amount of 0.2 to 23% by weight, or optionally further comprises polysaccharides in an amount of 20 to 25% by weight), and/or
comprises flavonoids in an amount of not more than 10% by weight (optionally comprises flavonoids in an amount of 0.05 to 5% by weight, optionally further comprises flavonoids in an amount of 0 to 2% by weight, optionally further comprises flavonoids in an amount of 0.05 to 2% by weight, optionally further comprises flavonoids in an amount of 0.5 to 1.5% by weight, optionally further comprises flavonoids in an amount of 0 to 1% by weight, or optionally further comprises flavonoids in an amount of 0.05 to 1% by weight), and/or
comprises amino acids in an amount of not more than 50% by weight (optionally comprises amino acids in an amount of 0 to 30% by weight, optionally further comprises amino acids in an amount of 0 to 25% by weight, optionally further comprises amino acids in an amount of 0 to 20% by weight, optionally further comprises amino acids in an amount of 0 to 5% by weight, optionally further comprises amino acids in an amount of 3 to 25% by weight, or optionally further extract comprises amino acids in an amount of 5 to 20% by weight), and/or
an additional component (optionally in an amount of 0 to 25% by weight, further optionally in an amount of 0 to 20% by weight, optionally in an amount of 0 to 15% by weight, optionally in an amount of 0 to 11% by weight, optionally in an amount of 2 to 20% by weight, or optionally in an amount of 4 to 8% by weight).

2. The use according to claim 1, wherein the male infertility is a male sexual dysfunction and/or a male reproductive dysfunction.

3. The use according to claim 1 or 2, wherein the male sexual dysfunction and/or the male reproductive dysfunction are caused by an endocrine abnormality and/or a metabolic abnormality; preferably, the metabolic abnormality is a glucolipid metabolic abnormality; preferably, the endocrine abnormality is a hormone level abnormality;
preferably, the glucolipid metabolic abnormality is at least one selected from diabetes, hyperglycemia, overweight, obesity, and hyperlipidemia.

4. The use according to any one of claims 1 to 3, wherein the male sexual dysfunction is at least one selected from libido disorders, erectile dysfunction, ejaculation disorders, and lack of orgasm; preferably, the male sexual dysfunction is erectile dysfunction and/or ejaculation disorders;
the male reproductive dysfunction is at least one selected from vas deferens obstruction, abnormal sperm quality, semen abnormality, varicocele, and inflammation; further preferably, the abnormal sperm quality is at least one selected from azoospermia, oligozoospermia, asthenozoospermia, and necrospermia; preferably, the male reproductive dysfunction is oligozoospermia, asthenozoospermia and/or oligoasthenozoospermia.

5. The use according to any one of claims 1 to 4, wherein the treatment and/or prevention of the male infertility specifically manifest in at least one of:
1) promoting spermatogenesis;
2) enhancing sperm motility;
3) improving sperm quality;
4) improving testicular index;
5) improving a structure of seminiferous tubules and a morphology and count of spermatogenic cells in testicular tissue;
6) improving a level of a relevant sex hormone; preferably, the sex hormone is androgen; further preferably, the androgen is testosterone;
7) improving an expression level of an androgen synthase;
8) improving an expression level of an androgen receptor; and
9) improving penile tissue injury and/or cell injury; preferably, the penile cells are endothelial cells of penile corpora cavernosa; preferably, improving a NO content in the endothelial cells of the penile corpora cavernosa; preferably, the improvement in the penile tissue injury comprises an improvement in at least one of an average thickness of cavernous scaffolds, a connectivity density, a degree of separation, and a mean intercept length of sinusoidal spaces;
further preferably, the treatment and/or prevention of the male reproductive dysfunction specifically manifest in at least one of:
1) promoting spermatogenesis;
2) enhancing sperm motility;
3) improving sperm quality;
4) improving testicular index;
5) improving a structure of seminiferous tubules and a morphology and count of spermatogenic cells in testicular tissue;
6) improving a level of a relevant sex hormone;
7) improving an expression level of androgen synthases;
8) improving an expression level of an androgen receptor; and
9) improving testicular cell injury; preferably, the testicular cells are testicular Leydig cells;
further preferably, the treatment and/or prevention of the male sexual dysfunction specifically manifest in at least one of:
1) improving a level of a relevant sex hormone; preferably, the sex hormone is androgen; further preferably, the androgen is testosterone;
2) improving an expression level of an androgen synthase;
3) improving an expression level of an androgen receptor;
4) improving penile tissue and/or cell injury; preferably, the penile cells are endothelial cells of penile corpora cavernosa; preferably, improving a NO content in the endothelial cells of the penile corpora cavernosa; preferably, the improvement in the penile tissue injury specifically comprises an improvement in at least one of an average thickness of cavernous scaffolds, a connectivity density, a degree of separation, and a mean intercept length of sinusoidal spaces; and
5) improving an IIEF-15 score; preferably, the IIEF-15 score includes at least one of erectile function, satisfaction with penetration, orgasm, libido, and overall satisfaction.

6. Use of a mulberry extract in preparation of a product for *in vitro* inhibition of high glucose-induced apoptosis of testicular Leydig cells;
optionally, use of a mulberry extract in a product for *in vitro* amelioration of high glucose-induced endothelial cell injury,
wherein, based on a weight percentage of a sum of components of the mulberry extract as 100%, the mulberry extract comprises alkaloids in an amount of 3% by weight or more (optionally comprises alkaloids in an amount of 3 to 99% by weight, further optionally comprises alkaloids in an amount of 15 to 99% by weight, further optionally comprises alkaloids in an amount of 30 to 99% by weight, further optionally comprises alkaloids in an amount of 40 to 99% by weight, further optionally comprises alkaloids in an amount of 50 to 99% by weight, or further optionally comprises alkaloids in an amount of 60 to 99% by weight), and/or
comprises polysaccharides in an amount of not more than 70% by weight (optionally comprises polysaccharides in an amount of 0.2 to 70% by weight, optionally further comprises polysaccharides in an amount of 0.2 to 50% by weight, optionally further comprises polysaccharides in an amount of 0.2 to 35% by weight, optionally further comprises polysaccharides in an amount of 0.2 to 25% by weight, optionally further comprises polysaccharides in an amount of 0.2 to 23% by weight, or optionally further comprises polysaccharides in an amount of 20 to 25% by weight), and/or
comprises flavonoids in an amount of not more than 10% by weight (optionally comprises flavonoids in an amount of 0.05 to 5% by weight, optionally further comprises flavonoids in an amount of 0 to 2% by weight, optionally further comprises flavonoids in an amount of 0.05 to 2% by weight, optionally further comprises flavonoids in an amount of 0.5 to 1.5% by weight, optionally further comprises flavonoids in an amount of 0 to 1% by weight, or optionally further comprises flavonoids in an amount of 0.05 to 1% by weight), and/or
comprises amino acids in an amount of not more than 50% by weight (optionally comprises amino acids in an amount of 0 to 30% by weight, optionally further comprises amino acids in an amount of 0 to 25% by weight, optionally further comprises amino acids in an amount of 0 to 20% by weight, optionally further comprises amino acids in an amount of 0 to 5% by weight, optionally further comprises amino acids in an amount of 3 to 25% by weight, or optionally further comprises amino acids in an amount of 5 to 20% by weight), and/or
an additional component (optionally in an amount of 0 to 25% by weight, further optionally in an amount of 0 to 20% by weight, optionally in an amount of 0 to 15% by weight, optionally in an amount of 0 to 11% by weight, optionally in an amount of 2 to 20% by weight, or optionally in an amount of 4 to 8% by weight).

7. The use according to any one of claims 1 to 6, wherein, based on the weight percentage of the sum of components of the mulberry extract as 100%, the weight contents of the components in the mulberry extract are as follows:
| | |
|---|---|
| alkaloids | 3-99%, |
| polysaccharides | 0.2-70%, |
| flavonoids | 0-10%, |
| amino acids | 0-50%, |
| additional component | 0-25%; |
optionally, based on the weight percentage of the sum of components of the mulberry extract as 100%, the weight contents of the components in the mulberry extract are as follows:
| | |
|---|---|
| alkaloids | 30-99%, |
| polysaccharides | 0.2-35%, |
| flavonoids | 0-2%, |
| amino acids | 0-30%, |
| additional component | 0-20%; |
optionally, based on the weight percentage of the sum of components of the mulberry extract as 100%, the weight contents of the components in the mulberry extract are as follows:
| | |
|---|---|
| alkaloids | 30-99%, |
| polysaccharides | 0.2-35%, |
| flavonoids | 0.05-2%, |
| amino acids | 0-25%, |
| additional component | 0-20%; |
optionally, based on the weight percentage of the sum of components of the mulberry extract as 100%, the weight contents of the components in the mulberry extract are as follows:
| | |
|---|---|
| alkaloids | 40-99%, |
| polysaccharides | 0.2-25%, |
| flavonoids | 0.05-1%, |
| amino acids | 0-25%, |
| additional component | 0-15%; |
optionally, based on the weight percentage of the sum of components of the mulberry extract as 100%, the weight contents of the components in the mulberry extract are as follows:
| | |
|---|---|
| alkaloids | 50-99%, |
| polysaccharides | 0.2-23%, |
| flavonoids | 0-1%, |
| amino acids | 0-20%, |
| additional component | 0-11%; |
optionally, based on the weight percentage of the sum of components of the mulberry extract as 100%, the weight contents of the components in the mulberry extract are as follows:
| | |
|---|---|
| alkaloids | 50-99%, |
| polysaccharides | 0.2-35%, |
| flavonoids | 0-2%, |
| amino acids | 0-30%, |
| additional component | 0-20%; |
optionally, based on the weight percentage of the sum of components of the mulberry extract as 100%, the weight contents of the components in the mulberry extract are as follows:
| | |
|---|---|
| alkaloids | 60-99%, |
| polysaccharides | 0.2-23%, |
| flavonoids | 0-1%, |
| amino acids | 0-5%, |
| additional component | 0-11%; |
further optionally, based on the weight percentage of the sum of components of the mulberry extract as 100%, the weight contents of the components in the mulberry extract are as follows:
| | |
|---|---|
| alkaloids | 50-65%, |
| polysaccharides | 20-25%, |
| flavonoids | 0.5-1.5%, |
| amino acids | 3-25%, |
| additional component | 2-20%; |
further optionally, based on the weight percentage of the sum of components of the mulberry extract as 100%, the weight contents of the components in the mulberry extract are as follows:
| | |
|---|---|
| alkaloids | 50-65%, |
| polysaccharides | 20-25%, |
| flavonoids | 0.5-1.5%, |
| amino acids | 5-20%, |
| additional component | 4-8%; |
further preferably, the alkaloids comprise one or more of 1-deoxynojirimycin (DNJ), N-methly-1-deoxynojirimycin, fagomine (FAG), 3-epi-fagomine, 1,4-dideoxy-1,4-imino-D-arabinitol (DAB), calystegin B2, calystegin C1, 2-O-(α-D-galactopyranosyl)-1-deoxynojirimycin, 6-O-(β-D-glucopyranosyl)-1-deoxynojirimycin, and 1,4-dideoxy-1,4-imino-(2-O-β-D-glucopyranosyl)-D-arabinitol;
optionally, a weight percentage of DNJ is not less than 50% (optionally 60 to 99%) of total alkaloids.

8. The use according to any one of claims 1 to 7, wherein
a method for preparing the mulberry extract comprises the following steps:
1) preparing a crude extraction solution of a plant of Moraceae; and
2) separating the crude extraction solution via a cation resin and/or an optional anion resin to obtain the mulberry extract;
preferably, the method further comprises the following steps:
3) subjecting effluent obtained from resin separation in step 2) to alcohol precipitation, and collecting a supernatant; and
4) subjecting the supernatant to concentration and/or drying;
optionally, the method further comprises the following step: subjecting effluent obtained from resin separation in step 2) to concentration and/or drying.

9. The use according to any one of claims 1 to 8, wherein the mulberry extract acts on humans or mammals.

10. The use according to any one of claims 1 to 9, wherein the drug is an oral dosage form; optionally, the drug is a tablet, a capsule, an oral solution, an oral emulsion, a pill, a granule, a syrup, and a powder.
